(19) Europäisches Patentamt / European Patent Office / Office européen des brevets

(11) EP 3 561 702 A1

(12) EUROPÄISCHE PATENTANMELDUNG

(43) Veröffentlichungstag:
30.10.2019 Patentblatt 2019/44

(51) Int Cl.:
G06F 19/00 (2018.01)        C08J 3/00 (2006.01)

(21) Anmeldenummer: 18168734.4

(22) Anmeldetag: 23.04.2018

(84) Benannte Vertragsstaaten:
AL AT BE BG CH CY CZ DE DK EE ES FI FR GB
GR HR HU IE IS IT LI LT LU LV MC MK MT NL NO
PL PT RO RS SE SI SK SM TR
Benannte Erstreckungsstaaten:
BA ME
Benannte Validierungsstaaten:
KH MA MD TN

(71) Anmelder: Covestro Deutschland AG
51373 Leverkusen (DE)

(72) Erfinder: Die Erfindernennung liegt noch nicht vor

(74) Vertreter: Levpat
c/o Covestro AG
Gebäude 4825
51365 Leverkusen (DE)

(54) **VERFAHREN ZUM ERMITTELN EINER PRODUKTKOMPOSITION FÜR EIN CHEMISCHES MISCHUNGSPRODUKT**

(57) Die Erfindung betrifft ein Verfahren zum Ermitteln einer Produktkomposition für ein chemisches Mischungssprodukt, wobei bei einer Vielzahl von ersten Produktkompositionen für ein jeweiliges chemisches Mischungsprodukt, wobei jede erste Produktkomposition durch eine numerische Produktverteilung zur Beschreibung von Anteilen von Komponenten der ersten Produktkomposition charakterisiert wird, für jede erste Produktkomposition der Vielzahl eine Reihe von Merkmalswerten, welche Merkmalswerte jeweils einen Deskriptor des jeweiligen Mischungsprodukts numerisch beschreiben, bereitgestellt wird, wobei die Reihe von Merkmalswerten für jedes Mischungsprodukt durch eine erste bijektive Abbildung auf eine Reihe von abgebildeten Merkmalswerten abgebildet wird, wobei für eine Vielzahl von zweiten Produktkompositionen für ein jeweiliges chemisches Mischungsprodukt eine Reihe von Prüfmerkmalswerten, welche Prüfmerkmalswerte jeweils eine Verhaltenseigenschaft des jeweiligen Mischungsprodukts numerisch beschreiben, bereitgestellt wird, wobei die Reihe von Prüfmerkmalswerten für jedes Mischungsprodukt durch eine zweite bijektive Abbildung auf eine Reihe von abgebildeten Prüfmerkmalswerten abgebildet wird, wobei zumindest die erste oder die zweite Abbildung eine Veränderung umfasst, wobei jede Reihe von abgebildeten Prüfmerkmalswerten einer zweiten Produktkomposition einer Reihe von veränderten Merkmalswerten einer ersten Produktkomposition zugeordnet wird, wobei durch eine multivariate Analyse der zugeordneten Reihen eine Korrelationsmatrix ermittelt wird, wobei ein Ziel-Anforderungsprofil zur Beschreibung mindestens einer Verhaltenseigenschaft eines Ziel-Mischungsprodukts vorgegeben wird und basierend auf dem Ziel-Anforderungsprofil sowie der Korrelationsmatrix ein Ziel-Deskriptorenprofil zur Beschreibung von Deskriptoren einer Ziel-Produktkomposition bestimmt wird.

Figur 1

EP 3 561 702 A1

**Beschreibung**

Die Erfindung betrifft ein Verfahren zum Ermitteln einer Produktkomposition für ein chemisches Mischungsprodukt, ein Verfahren zur Herstellung eines chemischen Mischungsprodukts aus einer Produktkomposition sowie ein chemisches Mischungsprodukt.

Kunststoffe werden heutzutage in nahezu zahllosen Anwendungen verwendet. Seit deren Entwicklung beginnend in der Mitte des 20. Jahrhunderts haben sich verschiedene Materialklassen- und technologien etabliert. Man unterscheidet dabei zwischen thermoplastischen Massenkunststoffen wie z.B. Polyethylen (PE), Polypropylen (PP), Polyester (PET, also Polyethylenglycolterephthalat, PBT, Polybutylenterephthalat), Polyvinylchlorid (PVC) und Thermosets wie z.B. Epoxyharze, Phenolharze, vulkanisierbare Gummimischungen, ungesättigte Polyester/Styrol-Mischungen, Silikone oder Polyurethane. Dabei haben die einzelnen Materialklassen jeweils eigene Märkte und Anwendungen sich erschließen können, was zumeist aufgrund ihrer hinreichenden Materialeigenschaften und Preispunkte nachvollziehbar ist.

Die ökonomische Seite beschränkt die Auswahl dieser Materialien, obwohl sogleich ein großer Bedarf an neuen Materialeigenschaften besteht. Dabei besteht zumeist nicht nur das Ziel eine spezifische Eigenschaftsanforderung zu erfüllen, sondern vielmehr ist der Wunsch eine Mehrzahl, oft mehr als drei Eigenschaften gleichzeitig zu erfüllen.

Die o.g. Thermoplaste sind für derartige Anforderungen meist nicht geeignet, da eine Produktanpassung in der Regel nicht zu den Skaleneffekten dieser Massenkunststoffe paßt. Aus diesem Grund wählt man für derartige Fälle Materialtechnologien aus, die eine inhärente Verfügbarkeit von miteinander kombinierbaren Bausteinen aufweisen, die zugleich die Kostenanforderungen erfüllen.

Weitverbreitete Systemchemien sind die Phenolharze, vulkanisierbare Gummimischungen, Silikone, Epoxysysteme und Polyurethane, die allesamt auch als Reaktivsysteme zur Verfügung stehen und in dieser Reihenfolge eine zunehmend größere Flexibilität der Einstellung von Eigenschaftsprofilen ermöglichen. Insbesondere sind es die Polyurethane, die eine enorme Anpassungsfähigkeit zeigen. Dies hat damit zu tun, dass es auf dieser Technologie vielfältige Isocyanate und isocyanatereaktive Komponenten gibt. Zudem gibt es diese auch als einkomponentige PU-Materialien, so dass hier von chemischen Mischungsprodukten gesprochen wird. Diese Vielfältigkeit stellt für den Entwickler eine besondere Chance aber eben auch eine Herausforderung dar, die es schwierig gestaltet, die beste Komponentenauswahl zu finden, insbesondere da auch Prozessbedingungen oftmals von Bedeutung sind. In der Industriepraxis hat sich hierfür ein iteratives, auf Versuch und Irrtum basierendes Vorgehen etabliert, wobei gerne mit einer schrittweisen Verbesserung ausgehend von einer naheliegenden Referenzkomposition begonnen wird. Diese Methodik weist aber mehrere Probleme auf, da hierbei Lokaloptima gefunden werden und weitere Alternativen grundsätzlich nicht berücksichtigt werden.

Ein weiteres Problem dabei ist, dass selbst wenn die erforderlichen Informationen zum Ermitteln einer geeigneten Produktkomposition im Grunde vorhanden sind, diese häufig bei unterschiedlichen Parteien vorliegen und schützenswerte Geschäftsgeheimnisse bilden. So kennt der Lieferant einer Produktkomposition als Ausgangsstoff deren Zusammensetzung. Der Abnehmer hingegen hat die Produktkomposition zur Herstellung eines Mischungsprodukts eingesetzt und kennt Prüfwerte des Mischungsprodukts. Für die betreffende Partei bzw. das betreffende Unternehmen liegt ein erhebliches Risiko darin, diese entsprechenden Informationen an die jeweils andere Partei ungefiltert weiterzugeben, da bspw. der Abnehmer dann die bekannte Produktkomposition von einem alternativen Lieferanten nachfragen kann. Umgekehrt könnte auch der Lieferant sensible Rückschlüsse aus den Prüfwerten ziehen und die entsprechenden Ergebnisse bei der Belieferung von Konkurrenten des Abnehmers berücksichtigen. In so einer Situation könnte das Teilen der entsprechenden Informationen also im Grunde zu der Ermittlung der richtigen Produktkomposition führen, was offenkundig für beide Seiten von kommerziellem Vorteil wäre, doch stellen die obenstehenden Gesichtspunkte wichtige Hemmnisse oder gar Ausschlusskriterien dar.

Aufgabe der vorliegenden Erfindung ist daher, eine gesuchte Produktkomposition für ein chemisches Mischungsprodukt ermitteln zu können, ohne dass es zu einer nachteiligen Verbreitung von sensiblen Daten kommt sowie ein solches chemisches Mischungsprodukt herstellen zu können.

Diese Aufgabe wird gelöst durch ein Verfahren zum Ermitteln einer Produktkomposition für ein chemisches Mischungsprodukt mit den Merkmalen des Anspruchs 1, durch ein Verfahren zur Herstellung eines chemischen Mischungsprodukts aus einer Produktkomposition mit den Merkmalen des Anspruchs 14 sowie durch ein chemisches Mischungsprodukt mit den Merkmalen des Anspruchs 15.

Der Erfindung liegt die Erkenntnis zugrunde, dass Daten sowohl der Produktkompositionen des Lieferanten als auch Prüfdaten der chemischen Mischungsprodukte des Abnehmers dann vergleichsweise problemlos herausgegeben werden können, wenn nicht nur die Zuordnung der Zahlenwerte zu entsprechenden Größen entfernt wird, sondern auch die Zahlenwerte selbst einer bijektiven Abbildung oder Funktion unterzogen werden.

Denn auch wenn die ausdrückliche Zuordnung zu bestimmten Größen nicht mitgeteilt wird, so ist doch regelmäßig für den Fachmann aus den Zahlenwerten selbst erkennbar, um was für eine Größe es sich handeln muss. Werden die Zahlenwerte selbst aber durch eine bijektive Funktion verändert, so entfällt diese Art der Erkennbarkeit, Korrelationszusammenhänge bleiben aber erhalten.

Das vorschlagsgemäße Verfahren dient dem Ermitteln einer Produktkomposition für ein chemisches Mischungsprodukt, wobei bei einer Vielzahl von ersten Produktkompositionen für ein jeweiliges chemisches Mischungsprodukt, wobei jede erste Produktkomposition durch eine numerische Produktverteilung zur Beschreibung von Anteilen von Komponenten der ersten Produktkomposition charakterisiert wird, für jede erste Produktkomposition der Vielzahl eine Reihe von Merkmalswerten, welche Merkmalswerte jeweils einen Deskriptor des jeweiligen Mischungsprodukts numerisch beschreiben, bereitgestellt wird. Die Produktkomposition ist in dem Sinne für ein chemisches Mischungsprodukt, dass die Produktkomposition ein Ausgangsstoff für das chemische Mischungsprodukt ist. Bevorzugt ist, dass jede Reihe von Merkmalswerten die gleiche Reihe von Deskriptoren beschreibt. Zwei oder mehrere Merkmalswerte, welche den gleichen Deskriptor beschreiben, können als typidentisch bezeichnet werden. Unter einem Deskriptor ist eine berechenbare, messbare oder sonstwie ermittelbare Eigenschaft einer Produktkomposition als Stoff zu verstehen. Eine solche Eigenschaft ist vorzugsweise unabhängig von einer weiteren Verarbeitung der Produktkomposition sein. Numerische Beschreibung bedeutet hier, dass die entsprechende Größe quantitativ - also durch eine Zahl - beschrieben wird.

Gemäß dem vorschlagsgemäßen Verfahren wird die Reihe von Merkmalswerten für jedes Mischungsprodukt durch eineerste bijektive Abbildung auf eine Reihe von abgebildeten Merkmalswerten abgebildet.

Weiter wird gemäß dem vorschlagsgemäßen Verfahren für eine Vielzahl von zweiten Produktkompositionen für ein jeweiliges chemisches Mischungsprodukt eine Reihe von Prüfmerkmalswerten, welche Prüfmerkmalswerte jeweils eine Verhaltenseigenschaft des jeweiligen Mischungsprodukts beschreiben, bereitgestellt. Bevorzugt ist, dass jede Reihe von Prüfmerkmalswerten die gleiche Reihe von Verhaltenseigenschaften beschreibt. Unter einer Verhaltenseigenschaft ist eine berechenbare, messbare oder sonstwie ermittelbare Eigenschaft einer Produktkomposition als Stoff zu verstehen. Bei einer solchen Verhalteneigenschaft kann es sich auch um Verarbeitungseigenschaften der Produktkomposition handeln.

Gemäß dem vorschlagsgemäßen Verfahren wird die Reihe von Prüfmerkmalswerten für jedes Mischungsprodukt durch einezweite bijektive Abbildung auf eine Reihe von abgebildeten Prüfmerkmalswerten abgebildet.

Vorschlagsgemäß umfasst zumindest die erste oder die zweite Abbildung eine Veränderung. Das bedeutet, dass zumindest die erste oder die zweite Abbildung eine Veränderung der abgebildeten Werte - also der Merkmalswerte bzw. der Prüfmerkmalswerte - umfasst. Mindestens eine der ersten oder der zweiten Abbildung ist daher keine Identitätsabbildung.

In dem vorschlagsgemäßen Verfahren wird jede Reihe von abgebildeten Merkmalswerten einer ersten Produktkomposition einer Reihe von abgebildeten Prüfmerkmalswerten einer zweiten Produktkomposition zugeordnet. Dies lässt sich auch dadurch ausdrücken, dass diese zweite Produktkomposition jener ersten Produktkomposition zugeordnet wird oder - was hier gleichbedeutend ist - umgekehrt.

Gemäß dem vorschlagsgemäßen Verfahren wird durch eine multivariate Analyse der zugeordneten Reihen eine Korrelationsmatrix zwischen den abgebildeten Merkmalswerten und den abgebildeten Prüfmerkmalswertenermittelt. Die Korrelationsmatrix kann auch Lücken umfassen, ist vorzugsweise aber vollständig. Vorzugsweise umfasst die Korrelationsmatrix Elemente, vorzugsweise Zahlen umfassend, welche Elemente qualitativ oder - wie bevorzugt - quantitativ eine jeweilige Korrelation zwischen den abgebildeten Prüfmerkmalswerten und den abgebildeten Merkmalswerten beschreiben. Der multivariaten Analyse der zugeordneten Reihen entspricht eine multivariate Analyse der abgebildeten Merkmalswerte und der abgebildeten Prüfmerkmalswerte.

Im vorschlagsgemäßen Verfahren wird ein Ziel-Anforderungsprofil zur Beschreibung von mindestens einer Verhaltenseigenschaft, vorzugsweise von mehreren Verhaltenseigenschaften, eines Ziel-Mischungsprodukts vorgegeben und basierend auf dem Ziel-Anforderungsprofil sowie der Korrelationsmatrix ein Ziel-Deskriptorenprofil zur Beschreibung von Deskriptoren einer Ziel-Produktkomposition bestimmt.

Wenn ein Ziel-Deskriptorenprofil, beispielsweise mit spezifischen Merkmalswerten für die Deskriptoren einer Ziel-Produktkomposition mit größerer oder geringerer Genauigkeit bekannt ist, kann die Produktverteilung für diese Ziel-Produktkomposition auf verschiedene Arten bestimmt werden, beispielsweise durch Rückgriff auf die Produktverteilungen von Produktkompositionen mit bekannten Merkmalswerten zur Beschreibung dieser Deskriptoren.

Daher ist es bevorzugt, dass das Bestimmen des Ziel-Deskriptorenprofils umfasst, basierend auf einem Vergleich des Ziel-Deskriptorenprofils mit den Merkmalswerten der ersten Produktkompositionen der Vielzahl eine erste Produktkomposition der Vielzahl als Ausgangs-Produktkomposition zu bestimmen und die Produktverteilung der Ausgangs-Produktkomposition basierend auf den Merkmalswerten der übrigen ersten Produktkompositionen der Vielzahl zum Erhalten der Ziel-Produktkomposition zu verändern.

Weitere Möglichkeiten und Weiterentwicklungen werden untenstehend beschrieben.

Der Begriff der bijektiven Abbildung kann auch als bijektive Funktion im mathematischen Sinne verstanden werden. Bijektiv bedeutet, dass die Abbildung bzw. Funktion sowohl surjektiv als auch injektiv ist.

Die Beschreibung einer Produktkomposition eines Mischungsproduktes durch eine numerische Verteilung - hier: Produktverteilung - von Anteilen der Komponenten der Produktkomposition folgt dem in der entsprechenden Industrie üblichen Inhaltsstoffen einer Formulierung:

Für Lacke sind dies üblicherweise ein oder mehrere Komponenten ausgewählt aus Bindemittel, wahlweise einem oder mehreren Vernetzern, Füller, Pigmente und Farbstoffe, Verlaufshilfsmittel, Entschäumer, Entgaser, Adhäsionsverbesserer, Stabilisatoren wie Antioxidantien und Lichtschutzmittel, Dispergier- und/oder Rheologieadditive, Oberflächenmodifikatoren, Lösemittel und/oder Wasser, Cosolventien, (Farb-)-Masterbatches, Initiatoren und/oder Katalysatoren, Corrosionschutzadditive.

Für Klebstoffe und Dichtmassen sind dies Bindemittel (z.B:.semikristalline Polymere für Schmelzklebstoffe, Dispersionen für wässrige Systeme), Lösemittel, Tackifier, Vernetzer, Katalysatoren, Weichmacher, Stabilisatoren, Thixotropiermittel, Beschleuniger sowie Farb- oder Füllstoffe.

Für Gießharze sind dies sind dies Bindemittel und Vernetzer, Flammschutzmittel, Katalysatoren, Weichmacher, Stabilisatoren, Beschleuniger sowie Farb- oder Füllstoffe und Additive.

Für Schäume sind dies Polyol und Isocyanat, Treibmittel, Wasser oder Luft, Katalysatoren, Stabilisatoren, Beschleuniger, Füllstoffe, Flammschutzmittel und Additive.

Die Beschreibung der Produktkomposition des Mischungsprodukts kann sich aber auch auf den chemischen Aufbau einer Einzelkomponente beziehen bzw. jene indirekt beschreiben. Dies Vorgehen bietet sich insbesondere an, wenn die Einzelkomponente optimiert werden soll. Mit Einzelkomponenten sind dabei die Komponenten eines Lackes, eines Klebstoffes, eines Gießharzes oder Schaums gemeint.

Dabei kann auf die Grundbausteine (Monomerkompositionen) und/oder die chemischen funktionellen Gruppen (z.B: Alkohol, Säure, Ether, Ester, Carbonat, Urethan, Harnstoff, Methylengruppe, aromatische oder aliphatische Ringstrukturen), die Molekulargewichtsverteilung, die Festkörpergehalte, Funktionalität oder Überstrukturen wie Hardsegmente, H-Brückenbildner, Haupt oder Seitenkettenzuordnung etc. Bezug genommen werden.

Die Produktverteilung von einer Produktkomposition ist nun die Mengenzusammensetzung der einzelnen Komponenten der Produktkomposition. Üblicherweise werden dabei bestimmte Mengenbereiche gewählt: Dabei bestimmen die Füller, Korrosionsschutzadditive und Pigmente einer Formulierung die Gesamtanteile und liegen bei 1-60% Gesamtanteil. Additive wie Verlaufshilfsmittel, Entschäumer, Entgaser, Adhäsionsverbesserer, Stabilisatoren wie Antioxidantien und Lichtschutzmittel, Dispergier- und/oder Rheologieadditive, Oberflächenmodifikatoren, Initiatoren und/oder Katalysatoren werden meist mit weniger als 5%, bevorzugt weniger als 1% eingesetzt. Lösemittel und Wasser setzt man bis zu 60% ein, um die Viskositäten einzustellen. Bindemittel und Vernetzer haben je nach Equivalentverhältnis zueinander ein festes Verhältnis und können in der Summe mit bis zu 50% (bei gefüllten Systemen) und bis zu 99% bei nicht gefüllten Systemen betragen.

Bei der chemischen Beschreibung der Komponenten kann die Produktbeschreibung noch detailreicher sein und betrifft dann die Gewichts-, Equivalent- oder molaren Verhältnisse der Grundbausteine, funktionellen Gruppen und/oder Überstrukturen.

Die Produktverteilung kann entweder aus Laborvorschriften bekannt sein oder ebenfalls auch durch experimentelle Methoden bestimmt werden. Insbesondere sind dabei Methoden geeignet, die entweder in der Lage sind absolute Anteile zu bestimmen, wie z.B. NMR-Spektroskopie oder aber auf Verfahren die relative Verhältnisse bestimmen lassen. Allgemein sind Verfahren wie die UV-, IR-, Raman-Spektroskopie, Massenspektrometrie, Gas, Flüssig-, Gelpermeations-chromatographische Verfahren aber auch Titrationsverfahren geeignet. Es ist auch möglich auf eine absolute bzw. durch Eichung abgeleitete Konzentration gänzlich zu verzichten. Dies ist insbesondere bei spektroskopischen/spektrometrischen Verfahren und chromatographischen Verfahren der Fall. Hier kann eine Kalibrierung mit den Features eines Chromatogramms und/oder Spektrogramm/Spektrums erfolgen, wobei dort zusätzlich digitale Trainingsverfahren (Neuronale Netze, Entscheidungsbäume o. drgl.) verwendet werden können.

Es ist möglich, dass eine erste Produktkomposition nicht identisch zu ihrer zugeordneten zweiten Produktkomposition ist. Vielmehr kann bevorzugt die eine Produktkomposition von der jeweils anderen abgeleitet sein. Es kann also sein, dass der Unterschied zwischen zwei zugeordneten Produktkompositionen aus einem jeweiligen Zusatz eines Stoffs besteht. Dieser zugesetzte Stoff kann aus einer Vielzahl von Substanzen in einer bestimmten Zusammensetzung bestehen. Der Zusatz kann in der ersten Produktkomposition gegenüber der zweiten Produktkomposition vorliegen oder umgekehrt. Vorzugsweise besteht ein konstanter und identischer Zusatz als Unterschied zwischen allen jeweils zugeordneten Produktkompositionen. Vorzugsweise beträgt dieser Zusatz weniger als 70 Gewichtsprozent, besonders bevorzugt weniger als 50 Gewichtsprozent und ganzbesonders bevorzugt weniger als 20 Gewichtsprozent bezogen auf die Gesamtkomposition.

Eine weitere bevorzugte Ausführungsform des Verfahrens ist dadurch gekennzeichnet, dass die Reihe von veränderten Prüfmerkmalswerten einer zweiten Produktkomposition derjenigen Reihe von veränderten Merkmalswerten einer ersten Produktkomposition zugeordnet wird, bei der die zweite Produktkomposition im Wesentlichen identisch zu der ersten Produktkomposition ist. Mit anderen Worten wird eine zweite Produktkomposition derjenigen ersten Produktkomposition zugeordnet, mit welcher sie im Wesentlichen identisch ist.

Gemäß einer bevorzugten Ausführungsform des Verfahrens ist vorgesehen, dass die Reihe von Merkmalswerten für jede erste Produktkomposition auf einem ersten Computersystem bereitgestellt wird, auf welchem die erste bijektive Abbildung ausgeführt wird, dass die Reihe von Prüfmerkmalswerten für jede zweite Produktkomposition auf einem zweiten Computersystem bereitgestellt wird, auf welchem die zweite bijektive Abbildung ausgeführt wird und dass das erste Computersystem und das zweite Computersystem von einem jeweils disjunkten Intranet umfasst sind. Damit erfolgt die Bereitstellung und Abbildung der Daten in jeweils getrennten Computersystemen. Anders ausgedrückt gibt es ein erstes Intranet, welches das erste Computersystem umfasst, und ein zweites Intranet, welches das zweite Computersystem umfasst, wobei das erste Intranet und das zweite Intranet keine Überschneidung aufweisen. Folglich müssen für eine datentechnische Verbindung zwischen dem ersten Intranet und dem zweiten Intranet beide Intranets verlassen werden, sodass eine solche Verbindung über ein - gegenüber den beiden Intranets - äußeres Netzwerk wie z. B. dem Internet erfolgen muss.

Gemäß einer weiteren bevorzugten Ausführungsform des Verfahrens ist vorgesehen, dass die Bestimmung des Ziel-Deskriptorenprofils zumindest teilweise auf dem ersten Computersystem durchgeführt wird.

Eine bevorzugte Ausführungsform des Verfahrens ist dadurch gekennzeichnet, dass die multivariate Analyse auf einem dritten Computersystem ausgeführt wird, welches von einem Intranet umfasst ist, dass zu dem jeweiligen Intranet des ersten Computersystems und des zweiten Computersystems disjunkt ist. In dieser Variante ist also ein gleichsam "neutrales" drittes Computersystem vorgesehen, welches sowohl gegenüber dem ersten Computersystem als auch gegenüber dem zweiten Computersystem getrennt ist. Auch ist es möglich, dass die multivariate Analyse auf dem ersten Computersystem und separat auf dem zweiten Computersystem durchgeführt werden und dann anschließend vergleichend diskutiert werden.

Eine weitere bevorzugte Ausführungsform des Verfahrens ist dadurch gekennzeichnet, dass die Produktverteilung und die Reihe von Merkmalswerten für jede erste Produktkomposition in dem ersten Computersystem gegenüber dem zweiten Computersystem datentechnisch abgekapselt wird und dass die Reihe von Prüfmerkmalswerten für jede zweite Produktkomposition in dem zweiten Computersystem gegenüber dem ersten Computersystem datentechnisch abgekapselt wird. Eine solche datentechnische Abkapselung bedeutet, dass in dem betreffenden System gegenüber dem abzukapselnden System technische Zugangshindernisse implementiert sind, welche einen aktiven Datenzugriff nur durch spezielle Autorisierung zulassen. Solche technischen Zugangshindernisse können auch gegenüber allen Zugriffen von außerhalb dem betreffenden Intranet gegenüber bestehen. Damit liegt nicht nur eine Trennung der Daten in verschiedene Computersystemen vor, sondern auch ein aktives Abschirmen der Daten voneinander.

Gemäß einer bevorzugten Ausführungsform des Verfahrens ist vorgesehen, dass die Reihe von abgebildeten Merkmalswerten für jede erste Produktkomposition von dem ersten Computersystem an ein Ziel-Computersystem eines disjunkten Intranets übertragen wird. Grundsätzlich kann es sich dabei um ein beliebiges Ziel-Computersystem sein. Bevorzugt ist, dass es an das zweite Computersystem oder das dritte Computersystem übertragen wird.

Bezüglich der Ermittlung einer Produktverteilung für die Ziel-Produktkomposition ist gemäß einer weiteren bevorzugten Ausführungsform des Verfahrens vorgesehen, dass ein Berechnungsmodell bereitgestellt ist, welches auf Eingabe von Merkmalswerten zur Beschreibung eines jeweiligen Deskriptors eine Produktverteilung einer Produktkomposition für ein Mischungsprodukt zur Approximation der Merkmalswerte ausgibt und dass das Ziel-Deskriptorenprofil dem Berechnungsmodell zur Ausgabe der Ziel-Produktkomposition eingegeben wird. Mit anderen Worten bietet das Berechnungsmodell die Möglichkeit, von gewünschten Merkmalswerten eine Produktverteilung zu bestimmen, deren Produktkomposition die gewünschten Merkmalswerte aufweist. Aus dem berechneten Zusammenhang mit den Verhaltenseigenschaften folgt dann, dass das chemische Mischprodukt auch die gewünschten Prüfmerkmalswerte aufweist. Bevorzugt erfolgt die Bereitstellung des Berechnungsmodells und alternativ oder zusätzlich die Eingabe des Ziel-Deskriptorenprofils in dem ersten Computersystem.

Grundsätzlich kann das Berechnungsmodell auf beliebige Art und Weise bestimmt worden sein. Eine hierauf aufbauende bevorzugte Ausführungsform des Verfahrens ist dadurch gekennzeichnet, dass das Berechnungsmodell zumindest teilweise durch, vorzugsweise in dem ersten Computersystem ausgeführte, multivariate Analyse der Reihe von Merkmalswerten jeder ersten Produktkomposition gegenüber der Produktverteilung dieser Produktkomposition ermittelt wird. Neben dieser multivariaten Analyse können auch analytische Überlegungen und Formeln in die Ermittlung des Berechnungsmodells fließen.

Grundsätzlich können die Merkmalswerte zur Beschreibung eines jeweiligen Deskriptors auf beliebige Art und Weise bestimmt werden. Eine weitere bevorzugte Ausführungsform des Verfahrens ist dadurch gekennzeichnet, dass für jede erste Produktkomposition der Vielzahl die Reihe von Merkmalswerten zumindest teilweise, vollzugsweise vollständig, durch eine Berechnung basierend auf der entsprechenden Produktverteilung ermittelt wird. Damit beruhen die Merkmalswerte nicht rein auf Messungen. Bevorzugt ist weiter, dass die Berechnung auf einem physikalischen Rechenmodell beruht, welches auf der Produktverteilung basiert. Dieses Rechenmodell kann auch eine teilweise oder vollständige Basis zur Ermittlung des obigen Berechnungsmodell bilden.

Die Errechnung der Merkmalswerte zur Beschreibung der jeweiligen Deskriptoren kann über verschiedene Verfahren erfolgen, bevorzugt sind stöchiometrische Verfahren, topologische und strukturelle Verfahren, physikochemische Verfahren, quantenchemische Verfahren oder spezielle Kennzahlen.

Unter stöchiometrischen Verfahren werden Methoden verstanden, die auf Basis von Gewichts/Equivalent/molaren Verhältnissen die Zusammensetzung von Grundbausteinen, funktionellen Gruppen, die Festkörpergehalte oder Überstukturen errechnen. Dabei werden Merkmalswerte für Deskriptoren erzeugt, wie z.B. die Urethandichte (in Gramm pro Kilogramm oder mol pro Kilogramm der Produktkomposition) oder der Anteil eines Monomers, wie z.B. die Menge eines verwendeten Isocyanats (in Gramm pro Kilogramm oder mol pro Kilogramm der Produktkomposition). Hierbei werden die Mengenanteile in allen Teilkomponenten bestimmt und dann auf die Gesamtmenge bezogen.

Unter topologischen und strukturellen Verfahren werden z.B. die Molekurgewichtsverteilung (massen- oder zahlengemittelt), die Netzwerkpunktdichte, die durchschittliche Netzbogenlänge oder deren Verteilung, der Anteil an Ringstrukturen oder anderer nicht vernetzter Anteile (Sol), der Gewichtsanteil an Hartsegment (als Teilmenge aus Diisocyanat und Diol sich bildender urethanreicher Substrukturen), bei phasenseparierter Materialien die entsprechenden Phasenanteile usw. gesehen. Hierzu können experimentelle Ergebnisse wie Molekulargewichtsverteilung durch GPC, theoretische Berechnungen wie Monte Carlo Simulationen, Röntgenstrukturmethoden zur Bestimmung kristalliner Anteile verwendet werden.

Unter physikochemische Verfahren werden Methoden zur Bestimmung zum Löslichkeitsverhalten, wie Oktanol-Wasser-Koeffizienten, Hansen-Löslichkeitsparameter, Dipolmomentbestimmung, Oberflächenladungseigenschaften (Zeta-Potential), Viskositäten, Oberflächenspannung (Tensiometrie), die in der Regel experimentell ermittelt oder durchgeführt werden verstanden. Es ist möglich die physikochemischen Methoden auf die einzelnen Komponenten zu beziehen. Ebenfalls ist es möglich diese auf strukturelle Subgruppen der Komponenten zu beziehen. Dabei ist dann eine Kombination von stöchiometrischen, topologischen und den physikochemischen Verfahren nutzbar, wobei dann die physikochemischen, experimentellen Verfahren auf Modellverbindungen angewendet werden und dann auf die strukturellen Subgruppen approximiert werden.

Unter quantenchemischen Verfahren werden Methoden zur Berechnung von Teilladungen, Polarisierbarkeit, Dipolmomenten, Orbitalenergien usw. verstanden, die mittels quantenchemischer Berechnungen durch ab-initio Methoden, Semiempirik.Methoden, Dichtefunktionaltheorie berechnet werden.

Spezielle Kennzahlen sind insbesondere solche, die aus einer chemischen Zusammensetzung oder Herstellung abgeleitet werden und dabei dem Fachmann eine direkte Verknüpfung zu seinem Handeln ermöglichen. Typische Beispiele bei Polyurethanen sind das Equivalentverhältnis von Isocyanat zu Alkohol, der Kettenverlängerungsgrad und Neutralisationsgrad bei Polyurethandispersionen oder Weichsegmentanteil, bei Epoxysystemen das Eq. Verhältnis zwischen Epoxy-Amin oder Epoxy-Säure, die Epoxyoligomerenverteilung, Epoxid-Bindemittelverhältnis usw.

Grundsätzlich kann es sein, dass die erste bijektive Abbildung jeden Eingangswert für sich und unabhängig von den anderen Eingangswerten abbildet. Mit anderen Worten ist dann jeder veränderte Merkmalswert nur von einem speziellen Merkmalswert vor der Veränderung abhängig. Alternativ oder zusätzlich kann dies auch für die zweite bijektive Abbildung bezogen auf die Prüfmerkmalswerte gelten. Gemäß einer bevorzugten Ausführungsform des Verfahrens ist jedoch vorgesehen, dass die erste bijektive Abbildung eine Koordinatentransformation von der Reihe von Merkmalswerten zu der Reihe von veränderten Merkmalswerten umfasst. Damit liegt eine erste bijektiive Abbildung vor, welche eine Reihe von Eingangswerten - die Merkmalswerte - auf eine ebenso große Zahl von Ausgangswerten abbildet, wobei dann grundsätzlich jeder Ausgangswert von einem, mehreren oder allen Eingangswerten abhängig sein kann und dabei insgesamt die Bijektivität gewahrt bleibt. In so einem Fall liegt eine Koordinatentransformation vor. Es ist weiter bevorzugt, dass alternativ oder zusätzlich und insbesondere sinngemäß gleich die zweite bijektive Abbildung eine Koordinatentransformation von der Reihe von Prüfmerkmalswerten zu der Reihe von veränderten Prüfmerkmalswerten umfasst.

Gemäß einer weiteren bevorzugten Ausführungsform des Verfahrens ist vorgesehen, dass die erste bijektive Abbildung und/oder die zweite bijektive Abbildung eine stetige und streng monotone Funktion mit sich kontinuierlich verändernder Ableitung ist.

Die Abbildung durch die bijektiven Abbildungen für alle Produktkompositionen erfolgt nach deren Zusammenstellung, wobei die Anordnung der Produktkompositionen jeweils gleich bleibt und somit vergleichbar zwischen den veränderten Merkmalswerten und den veränderten Prüfmerkmalswerten.

Beispiele für bijektive, stetige, streng monotone und wahlweise normierende Funktionen sind nachstehend beschrieben, wobei es sich bei den Werten um die Merkmalswerte oder die Prüfmerkmalswerte handeln kann

1. Bei nur positiven Werten, die Bestimmung des Kehrwerts des größten Werts aller betrachtenden Produktkompositionen und dann dessen jeweilige Multiplikation mit jedem Einzelwert oder alternativ zusätzlich die Multiplikation mit 100 (dann als Prozentwert).

2. Dem Quadrat des jeweiligen Werts und anschließender Multiplikation mit dem Kehrwert des Quadrats des größten Werts der betrachteten Produktkompositionen

3. Dem (dekadischen oder natürlichen) Logarithmus des Werts.

4. Der Wurzel des Werts.

5. Des Kehrwerts des Werts.

6. Die Bestimmung des arithmetischen Mittels und der Variation (Summe über den Quadraten der Differenzen aus Einzelwert minus arithmetischen Mittel - geteilt durch die Anzahl der betrachteten Werte). Die bijektive, stetige, streng monotone Funktion ist dann die Differenz des Werts minus des arithmetischen Mittels - geteilt durch die Varianz (studentische Verteilung).

Mit einer monotonen Funktion sind sowohl monoton steigende als auch monoton fallende Funktionen gemeint. Es können auch einzelne Werte monoton steigend und andere Werte monoton fallend gewandelt werden.

Es sind natürlich auch weitere bijektive, stetige, streng monotone und wahlweise normierende Funktionen denkbar. Diese können auf alle Werte - also jeweils Merkmalswerte oder Prüfmerkmalswerte - oder aber auch nur auf einzelne Werte angewendet werden. Auch ist es möglich für jede Reihe an Satz an beschreibenden Merkmalen oder Performancekriterien jeweils eine andere bijektive, stetige, streng monotone und wahlweise normierende Funktion anzuwenden.

Die erste bijektive Abbildung kann aus einer Reihe von jeweils deskriptorbezogenen Sub-Abbildungen bestehen, welche jeweils voneinander unabhängig sind. Hier ist bevorzugt, dass die erste bijektive Abbildung eine eindimensionale bijektive Sub-Abbildung für jeden einzelnen Deskriptor umfasst. Hier kann es sein, dass für jeden typengleichen Merkmalswert eine identische bijektive Sub-Abbildung erfolgt.

Entsprechend ist bevorzugt, dass die eindimensionale bijektive Sub-Abbildung jeden Deskriptors für jede erste Produktkomposition gleich ist.

Es ist aber alternativ auch möglich für die Merkmalswerte des gleichen Deskriptors unterschiedlicher erster Produktkompositionen eine jeweils andere bijektive Sub-Abbildung durchzuführen. Es ist auch möglich für Gruppen von Merkmalswerten jeweils die gleiche bijektive Sub-bbildung zu verwenden.

Sinngemäß gleich gilt dies auch für die zweite bijektive Abbildung. So ist es bevorzugt, dass die zweite bijektive Abbildung eine eindimensionale bijektive Sub-Abbildung für jede einzelne Verhaltenseigenschaft umfasst. Bevorzugt ist weiter, dass die eindimensionale bijektive Sub-Abbildung jeder Verhaltenseigenschaft für jede zweite Produktkompositon gleich ist. Sie können aber auch verschieden sein oder nur in Gruppen gleich sein.

Je nach den mathematischen Zusammenhängen der Merkmalswerte bzw. der entsprechenden Deskriptoren untereinander, der Prüfmerkmalswerte bzw. der Verhaltenseigenschaften untereinander und der Merkmalswerte bzw. der Deskriptoren zu den Prüfmerkmalswerten bzw. den Verhaltenseigenschaften zueinander eignen sich spezielle bijektive, stetige, streng monotone Funktionen besonders gut, was zu unterschiedlich hohen (also nahe bei 1,00) bzw. niedrigen (also nahe bei -1,00) Korrelationskoeffizienten führt. Daher ist es vorteilhaft mehrere unterschiedliche bijektive, stetige, streng monotone Abbildungen oder Funktionen auszutesten, um möglichst hohe Korrelationskoeffizienten zu erhalten, die eine Abhängigkeit der veränderten Merkmalswerte zu den veränderten Prüfmerkmalswerten unter- und zueinander besser beschreibt. Es wird bevorzugt, dass dann diese Korrelationskoeffizienten für die zu betrachtende Korrelationsmatrix verwendet werden.

Eine bevorzugte Ausführungsform des Verfahrens ist dadurch gekennzeichnet, dass die erste bijektive Abbildung eine jeweilige Normierungsfunktion für den jeweiligen Merkmalswert aufweist und/oder dass die zweite bijektive Abbildung eine jeweilige Normierungsfunktion für den jeweiligen Prüfmerkmalswert aufweist. Dabei kann es sein, dass die Ausgangsendpunkte der jeweiligen Normierungsfunktion der ersten bijektiven Abbildung durch die Spannbreite des jeweiligen Merkmalswerts für alle ersten Produktkompositionen bestimmt werden. Ebenso kann es sein, dass die Ausgangsendpunkte der jeweiligen Normierungsfunktion der zweiten bijektiven Abbildung durch die Spannbreite des jeweiligen Prüfmerkmalswerts für alle zweiten Produktkompositionen bestimmt werden.

Die jeweilige Normierungsfunktion kann wie für die bijektiven, stetigen, streng monotonen Funktionen gezeigt durch die Bestimmung des Kehrwerts des maximalen Werts für die jeweiligen Deskriptor oder die jeweilige Verhaltenseigenschaft für alle Produktkompositionen und dessen Multiplikation mit jedem einzelnen abzubildenden Wert für die jeweiligen Deskriptor oder die jeweilige Verhaltenseigenschaft erfolgen.

Weiterhin kann die optionale Normierung auch durch Bestimmung des maximalen $W_{max}$ und des minimalen Wertes $W_{min}$ aller Werte $W_i$ eines Deskriptors oder einer Verhaltenseigenschaft der Produktkompositionen erfolgen. Dabei wir von jedem Wert dieser minimale Wert subtrahiert und anschließend durch die Differenz von maximalen und minimalen Wert - was vorliegend auch als Spannbreite bezeichnet wird - geteilt, siehe Formel (1):

$$W_{i,norm} = (W_i - W_{min}) / (W_{max} - W_{min}) \qquad (1)$$

Durch die erste Abbildung beschreiben die abgebildeten Merkmalswerte nicht mehr direkt die Deskriptoren, sondern grundsätzlich ggf. nur fiktive Größen, welche durch die Anwendung des ersten Abbildung auf die Deskriptoren entstanden sind und hier als "abgebildete Deskriptoren" (Mi) bezeichnet werden. Aus den analogen Gründen beschreiben die abgebildeten Prüfmerkmalswerte die "abgebildeten Verhaltenseigenschaften" ($P_i$), wobei beide Größen nachfolgend der Einfachheit halber nur durch die Abkürzung referenziert werden.

Die obige multivariate Analyse der zugeordneten Reihen umfaßt zumindest eine Korrelationsberechnung, die es erlaubt in dem analysierbaren, multidimensionalen Raum (mit den Variablen der abgebildeten Deskriptoren $M_i$ und der abgebildeten Verhaltenseigenschaften $P_i$) auch die Abhängigkeiten der Verhaltenseigenschaften von den Deskriptoren durch die Berechnung des Korrelationskoeffizienten $r(M_j,P_j)$ zwischen $M_j$ und $P_j$ zu bestimmen. Dieser wird berechnet durch Formel (2)

$$r(M,P) = \frac{\sum_{i=1}^{n}(M_i-\bar{M})(P_i-\bar{P})}{\sqrt{\sum_{i=1}^{n}(M_i-\bar{M})^2}\sqrt{\sum_{i=1}^{n}(P_i-\bar{P})^2}} \qquad (2)$$

mit n der Anzahl der Produktkompositionen, $M_i$ den abgebildeten Deskriptoren M, $\bar{M}$ dem arithmetischen Mittel von M, $P_i$ den abgebildeten Verhaltenseigenschaften, $\bar{P}$ dem arithmetischen Mittel von P.

Als Teil der Ermittlung der Korrelationsmatrix können die einzelnen Korrelationskoeffizienten berücksichtigt, und in drei Kategorien eingeteilt werden:

1. $M_j$ korreliert positiv zu $P_j$, wenn $r(M_j,P_j) > c$,
2. $M_j$ korreliert negativ zu $P_j$, wenn $r(M_j,P_j) < -c$
3. $M_j$ korreliert nicht zu $P_j$, wenn $-c < r(M_j,P_j) < c$

wobei c der kritische Wert ist .(siehe auch A. L. Socklogg, J. N Edney, Some extension of Student's t and Pearson's r central distributions, Technical Report (May 1972), Measurement and Research Center, Temple University, Philadelphia). Für ein Vertrauensniveau von 0,05 (also 95%) ergibt sich beispielsweise bei n=11 ein c=0.602.

Bezogen auf ein Ziel-Anforderungsprofil, welches die gewünschten Verhaltenseigenschaften des insgesamt gewünschten Ziel-Mischungsprodukts beschreibt, werden nun die Verhaltenseigenschaften einzeln in ihrer Wunschgröße betrachtet. Werden in der Anwendung große Zahlenwerte gewünscht, so strebt man an die dazu positiv korrelierenden abgebildeten Merkmalswerte ebenfalls groß zu gestalten, bzw. die negativ korrelierenden werden dann klein gestaltet und die nicht korrelierenden können frei gewählt werden. Werden in der Anwendung kleine Zahlenwerte gewünscht, so strebt man an die dazu positive korrelierenden abgebildeten Merkmalswerte klein zu gestalten, bzw. die negativ korrelierenden werden dann groß gestaltet und die nicht korrelierenden können wiederum frei gewählt werden.

Es sei darauf hingewiesen, dass eine Verhaltenseigenschaft von einem oder mehreren Deskriptoren abhängen kann und diese ggf. auch andere Verhaltenseigenschaften bedingen. Auch können zwei Verhaltenseigenschaften von ein und denselben Deskriptor beinflußt sein. Hierbei gilt es also die Matrix der Abhängigkeiten aus der Korrelationsmatrix zu identifizieren und in der Planung mit aufzunehmen.

Es sollen im Folgenden verschiedenene Situationen und deren Lösung erläutert werden.

- $P_1$ und $P_2$ sollen beide möglichst groß (oder einfach größer) werden und $M_1$ korreliert mit beiden positiv. In diesem Fall sollte(n) die neu(en) zu prüfenden Komposition(en) derart gewählt werden, in denen $M_1$ ebenfalls größer wird.
- $P_1$ und $P_2$ sollen beide möglichst klein (oder einfach kleiner) werden und $M_1$ korreliert mit beiden positiv. In diesem Fall sollte(n) die neu(en) zu prüfenden Komposition(en) derart gewählt werden, in denen $M_1$ ebenfalls kleiner wird.
- $P_1$ und $P_2$ sollen beide möglichst groß (oder einfach größer) werden und $M_1$ korreliert mit beiden negativ. In diesem Fall sollte(n) die neu(en) zu prüfenden Komposition(en) derart gewählt werden, in denen $M_1$ ebenfalls kleiner wird.
- $P_1$ und $P_2$ sollen beide möglichst klein (oder einfach kleiner) werden und beschreibendes $M_1$ korreliert mit beiden negativ. In diesem Fall sollte(n) die neu(en) zu prüfenden Komposition(en) derart gewählt werden, in denen $M_1$ ebenfalls größer wird.
- Einer der beiden von $P_1$ und $P_2$ soll möglichst groß (oder einfach größer) und der andere möglichst klein (oder kleiner) werden und $M_1$ korreliert mit beiden positiv oder negativ. Wenn $P_1$ und $P_2$ keine weiteren positiven oder negativen Korrelationen aufweisen, bietet es sich an neue, andere Deskriptoren zu entwickeln, die aus wissenschaftlicher Sicht mit $M_1$ verwandt sind, selbst aber hinreichen unterschiedlich erscheinen. Das Ziel es ist, die neuen Deskriptoren $M_{1-A}$, $M_{1-B}$, ... (also abgeleitete Eigenschaften von $M_1$) so zu wählen, dass zumindest eines davon nur mit $P_1$ bzw. $P_2$ positiv oder negativ korreliert.
- $P_1$ soll möglichst groß (oder einfach größer) und $P_2$ möglichst klein (oder kleiner) werden und $M_1$ korreliert mit beiden positiv. Wenn $P_1$ eine weitere positive Korrelationen $M_2$ aufweist, bietet es sich an die neu(en) zu prüfenden Komposition(en) derart zu wählen, dass $M_2$ ebenfalls größer wird, $M_1$ dagegen kleiner wird.

Wie aus den Beispielen klar wird, hilft die Korrelationsmatrix die Abhängigkeiten zu identifizieren und damit eine Handlungsanweisung zu bekommen, wie Verhaltenseigenschaften verbessert werden können. Wenn Kreuzabhängigkeiten existieren, kann eine Balance (wie im letzten Spiegelstrich gezeigt) gesucht werden. Sind die Abhängigkeiten derart, dass das Ziel-Anforderungsprofil nicht erreicht werden kann, gilt es die Deskriptoren differenzierter auszuarbeiten, in der Hoffnung dann eine Korrelationsmatrix zu erhalten, die dann eine erneute Handlung entsprechend erlaubt.

Weiterhin können auch die Korrelationskoeffizienten zwischen den Deskriptoren oder den $M_i$ bzw. zwischen den Verhaltenseigenschaften oder den $P_i$ selbst betrachtet werden. Werden dort hohe signifikante Werte (also größer dem kritischen Wert oder kleiner dem Negativen des kritischen Wertes) gefunden, kann dies auch zur Dimensionsreduktion genutzt werden. Somit erkennt man sehr schnell welche Deskriptoren miteinander bedingen und können damit auch helfen, selbige vollständiger zu verstehen. Als Beispiel seien die beiden Deskriptoren "Urethandichte" und "Isocyanatmonomergehalt" genannt, die - je nach Design der Studie - häufig direkt miteinander korrelieren, da Urethane aus einem Isocyanat gebildet werden.

Alternative Verfahren zur Abhängigkeitsermittlung sind ebenfalls möglich, insbesondere sei hier die Hauptkomponentenanalyse (Principle Component Analysis, PCA) genannt (siehe hierzu auch G. H. Dunteman: Principal Component Analysis. Sage Publications, 1989). Dies ist ein mathematisches Verfahren, das eine Koordinatentransformation=Hauptachsentransformation mit orthogonalen Achsen durchführt und zwar als Kriterium gilt hier, dass die erste Hauptkomponente (mathematisch formuliert als Linearkombination der einzelnen Merkmale) die größte Varianz aufweist und die zweite Hauptkomponente die zweitgrößte Varianz aufweist. In der Analyse wird dann geprüft, für welche Deskriptoren oder $M_i$ bzw. welche Verhaltenseigenschaften oder $P_i$ selbst hohe Abhängigkeiten bestehen und damit die Korrelationsmatrix reduziert werden kann bzw. welche Deskriptoren bzw. $M_i$ und Verhaltenseigenschaften bzw. $P_i$ Paare bilden, von denen ein Paarmitglied zur Analyse der Korrelationsmatrix in der multivariate Analyse entfernt werden kann.

Es ist auch möglich, daß verschiedene bijektive Funktionen geprüft werden, wobei dann für ein gegebenes Paar aus Deskriptor bzw. $M_i$ und Verhaltenseigenschaft bzw. $P_i$ unterschiedlich hohe (für positive Korrelation) und niedrige (für negative Korrelation) Werte erhalten werden. Es ist bevorzugt dann die maximal positiven bzw. minimalen negativen Werten zur Auswertung in der multivariaten Analyse zu verwenden.

Zur Berechnung der Komposition des Ziel-Mischungsprodukts - also der Ziel-Produktkomposition - wird die Veränderlichkeit der Prüfmerkmalswerte mit Produktkompositionsänderungen analysiert. Dabei wird vorzugsweise eine Einteilung der zur Verfügung stehenden Kompositionen durchgeführt, wobei gleiche Veränderungsschemata verwendet werden. Dies können z.B. die systematische Änderungen der Anteile von Einzelkomponenten sein, die Änderung von chemischen Equivalenten oder andere inkrementelle Produktkompositionsänderungen. Für diese Serien mit gleichem Veränderungsschemata erfolgt dann eine Betrachtung der Prüfmerkmalswerte. Anschließend kann man von jeder beliebigen verfügbaren Komposition bei einer derartigen systematischen Änderung an jeder verfügbaren Komposition die Änderung der Eigenschaft in einem begrenzten Umfeld vorhersagen. Für die Berechnung der Ziel-Produktkomposition des Ziel-Mischungsprodukt wird nun der in der multivariaten Analyse identifizierte korrelierende oder determinierende Deskriptor ausgewählt und dessen Merkmalswert bestimmt, der dann dem Prüfmerkmalswert des Ziel-Mischungsprodukts entspricht. Dies kann z.B. mit einer grafischen Darstellung einfach erfolgen, in dem man die Prüfmerkmalswerte gegen den determinierenden Deskriptor aufträgt und dann mit der Veränderlichkeit der Prüfmerkmalswerte (also der Steigung in der Kurve) ausgehend von der neuen Referenzposition (also einer der verfügbaren Kompositionen) mit gleicher Steigung abträgt. Bei Erreichen des Prüfmerkmalwertes für die Verhaltenseigenschaft gemäß Ziel-Anforderungsprofil kann der Wert des Deskriptors abgelesen werden, wodurch das Ziel-Deskriptorenprofil aufgebaut werden kann. Mit diesem Wert wird dann die Komposition des Ziel-Mischungsprodukts im Computer unter Variation des betrachteten Veränderungsschematas eingestellt und die Ziel-Produktkomposition bestimmt.

Alternativ, insbesondere bei einer Vielzahl von Produktkompositionen die mit keinem systematischen Veränderungsschemata variiert worden sind, kann auf die Einteilung nach Veränderungsschemata verzichtet werden und alle Datensätze von Produktkompositionen genutzt werden. In diesem Fall ist es oft vorteilhaft die Veränderlichkeit der Prüfmerkmale als Funktion von mehr als einem determinierenden Deskriptor zu beschreiben.

Eine bevorzugte Ausführungsform des Verfahrens ist dadurch gekennzeichnet, dass das chemische Mischungsprodukt ein Phenolharz, eine vulkanisierbare Gummimischung, ein Silikon, ein Epoxysystem und/oder einen festen Schaumstoff, insbesondere ein Polyurethan, umfasst oder daraus besteht.

Im Folgenden soll nun für die verschiedenden chemischen Mischungsprodukte Epoxyharze, Phenolharze, vulkanisierbare Gummimischungen, ungesättigte Polyester/Styrol-Mischungen, Silikone oder Polyurethane Deskriptoren zur Beschreibung durch Merkmalswerte genannt werden, die in Produktentwicklungen mit solchen Materialtechnologien besonders geeignet sind.

Allgemein für alle chemischen Mischungsprodukte anwendbar sind folgende Deskriptoren als Gehalt (als Equivalentgewicht oder Gewichtsprozentangabe): Kohlenstoffgehalt, Wasserstoffgehalt, Sauerstoffgehalt, Stickstoffgehalt, Siliziumgehalt, Schwefelgehalt, Halogengehalt, Knotenpunktdichte (als Equivalentgewicht)

Als Deskriptoren für vulkanisierbare Gummimischungen besonders geeignet sind (als Equivalentgewicht oder Gewichtsprozentangabe) Methylengruppen, Methylseitengruppen, nicht aktivierte Doppelbindungen, aktivierte Doppelbindungen, Styrol, Schwefelgehalt, Vernetzungspunkte, Mercaptobenzothiazolgehalt, Dimethyldithiocarbaminsäuregehalt, Tetramethylthiuramdisulfidgehalt, Vulkanisationszeit und -temperatur.

Als Deskriptoren für Silikone besonders geeignet sind (als Equivalentgewicht oder Gewichtsprozentangabe) Dimethylsilanoxygruppengehalt, Diphenylsilanoxygruppengehalt, Methylphenylsilanoxygruppengehalt, Monomethylsilanoxygruppengehalt, Monophenylsilanocygruppengehalt, Trimethylsilanoxygruppengehalt, Silikonacrylatgruppengehalt, Titantetrabutanolatgehalt, Silangruppengehalt, Allylsilylgruppengehalt, Aminopropylsilygruppengehalt.

Als Deskriptoren für Epoxide und Phenolharze besonders geeignet sind (als Equivalentgewicht oder Gewichtsprozentangabe): Bisphenol-A-Epoxidgehalt, Bisphenol-F-Epoxidgehalt, Tetraglycidylethergehalt des Tetraphenyle-

thans, Glycidyloxphenylenmethylengruppengehalt (von Phenolnovolackharze), Glycidyloxtolylenmethylengruppenge-halt (von Kresolnovolackharze), Epoxidgruppengehalt, Napthyldiglycidiylgruppengehalt, Katalysatorengehalte wie z.B. Tetraalkylammoniumsalze, Lithiumbromid, Cholinchlorid, Imidazole, Triglycidylcyanuratgruppen, Equivalentverhältnisse wie Epoxid zu Amin, Epoxid zu Carboxylgruppenverhältnisse.

Als Deskriptoren für Polyurethane besonders geeignet sind (als Equivalentgewicht oder Gewichtsprozentangabe): Urethangruppengehalt, Harnstoffgruppengehalt, Biuretgruppengehalt, Isocyanuratgruppengehalt, Allophanatgruppengehalt, Estergruppengehalt, Ethergruppengehalt, Carbonatgruppengehalt, Carboxylgruppengehalt, Carbonylgruppengehalt, Sulfonsäuregruppengehalt, OH-Gruppengehalt, NCO-Gruppengehalt (z.B. für Prepolymere), H-Donorgruppengehalt (als Equivalentgewicht), H-Akzeptorgruppengehalt (als Equivalentgewicht), Knotenpunktdichte (als Equivalentgewicht), Hartsegmentanteil (als Gewichtsanteil, definiert als Anteil aller urethanisierter Diole),

Gewichtsanteile üblicher Isocyanatkomponenten, wie z.B. 1,4-Butandiisocyanat, 1,5-Pentandiisocyanat, 1,6-Hexandiisocyanat (Hexamethylendiisocyanat, HDI), 2,2,4-Trimethylhexamethylen¬di¬iso¬cyanat und / oder 2,4,4-Trimethylhexamethylendiisocyanat (TMDI), Isophorondiisocyanat (IPDI), Bis-(4,4'-isocyanatocyclo¬ihexyl)methan und / oder Bis-(2',4-isocyanatocyclo¬hexyl)methan, , 2,4- und / oder 2,6-Toluylendiisocyanat (TDI), 1,5-Naphthylendiisocyanat (NDI), 2,4'- und / oder 4,4'-Diphenylmethandiisocyanat (MDI), 1,3-Bis(isocyanatomethyl)benzol (XDI) und / oder das analoge 1,4-Isomere,

Gewichtsanteile üblicher Alkoholkomponenten oder Ethervorstufen, wie z.B. Ethylenglycol, Propylengylcol, auch Ethylenoxid, Propylenoxid, Butandiol, Neopentlygylcol, Hexandiol, Trimethylolpropan, Glycerin, Pentaerythrit, Zucker und deren Derivate (z.B. Sorbit),

Gewichtsanteile üblicher Säuren in Polyestern, wie z.B. Adipinsäure, Terepthalsäure, Isophtalsäure, Pthalsäure (anhydrid), Trimellithsäure (anhydrid), Bernsteinsäure, Korksäure, Sebacinsäure, Decandicarbonsäure,

Gewichtsanteiler üblicher Monomere in Polyacrylatbausteinen, wie z.B. Methylacrylat, Methylmethacrylat, Ethylacrylat, Ethylmethacrylat, Ethoxyethylacrylat, Ethoxyethylmethacrylat, n-Butylacrylat, n-Butylmethacrylat, tert.-Butylacrylat, tert.-Butylmethacrylat, Hexylacrylat, Hexylmethacrylat, 2-Ethylhexylacrylat, 2-Ethylhexylmethacrylat, Butoxyethylacrylat, Butoxyethylmethacrylat, Laurylacrylat, Laurylmethacrylat, Isobornylacrylat, Isobornylmethacrylat, Phenylacrylat, Phenylmethacrylat, Phenoxyethylacrylat, Phenoxyethylmethacrylat, Phenoxyethoxyethylacrylat, Phenoxyethoxyethylmethacrylat, Bisphenol A Diacrylat, Bisphenol A Dimethacrylat, 2-Hydroxyethyl(meth)acrylat, 2-Hydroxypropyl(meth)acrylat, 4-Hydroxybutyl(meth)acrylat, Stearylacrylate, die alle auch in anderen Acrylatlacksystemen wie z.B. Polyacrylatdispersionen Anwendung finden,

Equivalentverhältnisse von Isocyanat zu Alkohol, Isocyanat zu Amin und Isocyanat to Summe der isocyanatreaktiven Gruppen.

Als Deskriptoren für strahlenhärtende Systeme eignen sich insbesondere die Gewichtsanteile üblicher höherfunktioneller Reaktivverdünner, wie z.B: Hexandioldiacrylat, Butandioldiacrylat, Di- und triproyplenglycoldiacrylat, Bisphenol A- diacrylat, Trimethylolpropantriacrylat, Pentaerythroltriacrylat, Trimethylolpropantetraacrylat, Dipentaerythrolpenta- und hexaacrylat, Benzylmethacrylat, Isodecylmethacrylat, Ethylenglycoldiacrylat, Bisphenol A epoxyacrylat, Phosphatemethacrylat, Carboxethylacrylat.

Als Deskriptoren für Hybridsysteme, die Strukturelemente der beschriebenen Materialtechnologien Epoxyharze, Phenolharze, vulkanisierbare Gummimischungen, ungesättigte Polyester/Styrol-Mischungen, Silikone oder Polyurethane vereinen, bzw. in Produktkombinationen gemeinsam auftreten ist es natürlich auch möglich die o.g. Deskriptoren zu kombinieren, so können beispielsweise in Isocyanate modifizierten Epoxysystemen neben den besonders für Epoxide geeigneten Deskriptoren auch jene mit denen der für Polyurethane geeignete Deskriptoren verwendet und gemeinschaftlich in dem vorschlagsgemäßen Verfahren genutzt werden.

Weiterhin sollen nun beispielhafte Verhaltenseigenschaften genannt werden, die in Lack- und Klebstoffanwendungen typischerweise Teil eines Ziel-Anforderungsprofils sind und damit als Prüfmerkmalswerte $P_i$ von Verhaltensei-genschaften zur Anwendung kommen. Hierbei unterscheidet man in Lackeigenschaften wie Flammpunkt, Topfzeit, Trocknungszeit, Viskosität, die Oberflächenspannung, Abrasionsfestigkeit, Ergebnisse aus der Kapillarmethode, dem Blasendrucktensiometer, Oberflächeneigenschaften des gehärteden / getrockneten Lackes, wie z.B., Glanz, Farbe, Härte, Kratzfestigkeit, Einkerbung, Flexibilität, Benetzungsstörungen wie Entnetzung, Kraterbildung, Fischaugen, Benardzellen, Orangenhaut, Oberflächenrauheit, gleitende Eigenschafte, Effektoberflächeneigenschaften wie Strukturlacke, Hammerschlageffekte,

Bulkeigenschaften des gehärteden / getrockneten Lackes, Filmdicke, Deckkraft, wie z.B. Hansenparameter, Viskosität der Komposition (in Abhängigkeit von Temperatur, Scherrate), Strukturviskosität, Thixotropie, Dilatanz, Rheopexie, mechanische Eigenschaften wie Elastizität und Dehnbarkeit (z.B. die lineare elastische Deformation, plastische Deformation, Streckspanung, Streckgrenze, Bruchspannung, Bruchdehnung), aber auch Gesamtsystemprüfungen wie Wasserdampfbeständigkeit, Wasserfestigkeit, Lösemittelbeständigkeit, Korrosionsbeständigkeit, Bewitterungsfähigkeit, biologischer Abbau durch Bakterien, Befall von Pilzen, Algen oder Seepocken,

Adhäsionseigenschaftem des gehärteten / getrockneten Lackes, wie z.B. die Adhäsionskraft bei einem Schälversuch unter Adhäsionsverlust, das Ergebnis eines Gitterschnittversuchs, und Adhäsionsbeständigkeit.

Bei Klebstoffen sind die Zugspannung, Abzugsprüfungen, Scherbelastung, Schälprüfung, Biegeprüfung, Schlagfestigkeit, Bruchverhalten wie Kohäsions- oder Adhäsionsbruch, Bruchbildprüfung, Oberflächenspannung, Tempertur und Feuchtigkeitsbeständigkeit, Soforthaftung, Langzeithaftung, Aushärtezeit geeignete Verhaltenseigenschaften

Das vorschlagsgemäße Verfahren ist besonders geeignet für die Entwicklung von Produktkomposition und deren Produktverteilungen von Thermoplasten, Thermosets, 2-Komponenten Reaktivmaterialen, Lacken, Klebstoffen, Gießmassen, Schaumstoffen, dabei besonders bevorzugt für Lacke und Klebstoffe. Insbesondere ist es auch geeignet für Epoxysysteme, Phenolharzsysteme, vulkanisierbare Gummimischungen, ungesättigte Polyester/Styrol-Mischungen, Silikonsystem oder Polyurethansystem, wobei besonders bevorzugt Epoxysysteme, Silikonsystem oder Polyurethansystem geeignet sind und ganz besonders bevorzugt Polyurethansysteme sind.

Das vorschlagsgemäße Verfahren zur Herstellung eines chemischen Mischungsprodukts aus einer Produktkomposition ist dadurch gekennzeichnet, dass die Produktkomposition nach dem vorschlagsgemäßen Verfahren zum Ermitteln einer Produktkomposition für ein chemisches Mischungsprodukt ermittelt wurde.

Das vorschlagsgemäße chemische Mischungsprodukt ist dadurch gekennzeichnet, dass das chemische Mischungsprodukt durch das vorschlagsgemäße Verfahren zur Herstellung eines chemischen Mischungsprodukts hergestellt wurde.

Bevorzugte Ausführungsformen und Varianten des vorschlagsgemäßen Verfahrens zur Herstellung eines chemischen Mischungsprodukts sowie des vorschlagsgemäßen chemischen Mischungsprodukts entsprechend bevorzugten Ausführungsform und Varianten des vorschlagsgemäßen Verfahrens zum Ermitteln einer Produktkomposition und umgekehrt.

Weitere Einzelheiten, Merkmale, Ausgestaltungen, Ziele und Vorteile der vorliegenden Erfindung werden nachfolgend anhand der Zeichnung erläutert. In der Zeichnung zeigt

Fig. 1    beispielhaft eine starke positive Korrelation des Harnstoffgehalts als Deskriptor mit dem Elastizitätsmodul als Verhaltenseigenschaft,

Fig. 2    beispielhaft eine mit der Korrelation der Fig. 1 einhergehende negative Korrelation der Bruchdehnung als Verhaltenseigenschaft,

Fig. 3    beispielhaft die Ableitung der Veränderung des Elastizitätsmoduls als Verhaltenseigenschaft zwischen zwei Produktkompositionen und die Prognose der Veränderung des Elastizitätsmoduls bei einer veränderten Produktkomposition,

Fig. 4    beispielhaft die Ableitung der Veränderung der Bruchdehnung als Verhaltenseigenschaft zwischen den beiden Produktkompositionen der Fig. 3 und die Prognose der Veränderung des Bruchdehnung bei der veränderten Produktkomposition der Fig. 3 und

Fig. 5    drei Computersysteme zur Ausführung eines Ausführungsbeispiel des vorschlagsgemäßen Verfahrens zum Ermitteln einer Produktkomposition für ein chemisches Mischungsprodukt.

Im Folgenden wird die Erfindung auch anhand von Tabellen im Detail erläutert. Es zeigen:

Tabelle A verschiedene Polyurethane als Produktkompositionen mit Produktverteilungen entnommen aus W. Panwiriyarat et al., J. Polym. Environ. 21, 807-815 (2013), Table 1, S. 809 oben. Dabei sind die molaren Verhältnisse der vier Kompositionsbestandteile angegeben: IPDI: Isophorondiisocyanat mit eine Molmasse von 220; PCL: Polycaprolactondiol hergestellt aus Ethylenglycol und Caprolacton mit einer Molmasse von 530; HTNR: ein ungesättigtes Kautschukdiol mit einer Molmasse von 1700 mit durchschittlich 23,6 Doppelbindungen und BDO: Butandiol mit einer Molmasse von 90.

Tabelle B zeigt die Verhaltenseigenschaften der Produkte aus Tabelle A, entnommen aus W. Panwiriyarat et al, J. Polym. Environ. 21, 807-815 (2013), Tabelle 2, S. 811 mit dem Elastizitätmodul in MPa ("YoungMod"), der Bruchkraft in Mpa ("TensileStr"), der Bruchdehnung in Prozent ("EaB"), der Reißfestigkeit in N/mm$^2$ ("TearStr") und der Shore A Härte - einheitenfrei - ("Shore A")

Tabelle 1a: Für die Polyurethane 3 bis 14 die Deskriptoren: Hardsegmentanteil in Gewichtsprozent ("HardSeg"), Harnstoffanteil in Equivalent/kg ("Urea"), Urethananteil in Equivalent/kg ("Urethane"), Esteranteil in Equivalent/kg ("Ester"), Doppelbindungsanteil in Equivalent/kg ("Doublebond"), Butandiolanteil in Equivalent /kg ("BDO") und die Performanceeigenschaften $P_i$: das Elastizitätmodul in Mpa ("YoungMod"), die Bruchkraft in Mpa ("TensileStr"), die Bruchdehnung in Prozent ("EaB"), Reißfestigkeit in N/mm$^2$ ("TearStr") und die Shore A Härte - einheitenfrei - ("Shore A")

Für die Berechnung des Hardsegmentanteils wurden die Gewichtsanteile alle Diisocyanate (hier IPDI) und Diole (hier Butandiol) zum Gesamtgewicht bezogen/berechnet und in Prozent angegeben.

Für die Berechnung des Harnstoffanteil wurden der Isocyanatüberschuß der nicht mit Alkoholgruppen abreagierten Isocyanatgruppen errechnet. Diese reagieren mit Umgebungswasser zu Carbaminsäure und unter Kohlendioxidabspaltung zu Amin, dass mit einem weiteren Isocyanat zu Harnstoff schnell abreagiert. Somit entstehen aus zwei überschüssigen Isocyanatgruppen eine Harnstoffgruppe. Die molare Menge dieser Harnstoffgruppen wird dann auf ein Kilogramm Gesamtprodukt bezogen/berechnet.

Die Berechnung des Urethananteils errechnet sich aus den hier im Unterschuß sich befindenden Alkoholgruppen. Jede reagiert zu einer Urethangruppe ab, so daß die molare Menge der Alkoholgruppen pro Kilogramm Gesamtprodukt den Urethananteil in Equivalent/kg ergibt.

Die Berechnung des Esteranteils erfolgt durch die im Polycaprolacton ("PCL") enthaltenen Estergruppen von 2,05 Estergruppen pro Equivalent Polycaprolacton von 265 g. Somit errechnet sich der Esteranteil aus der Menge an Esterequivalenten in der verwendeten Menge des Polycaprolactons bezogen auf einem Kilogramm Gesamtprodukt.

Die Berechnung des Doppelbindungsanteils erfolgt durch die im ungesättigten Polybutadiendiols ("HTNR") enthaltenen Doppelbindungen von 23,6 Doppelbindungsgruppen pro Molgewicht von 1700g/mol HTNR, bei einem Equivalentgewicht von 850 g. Somit errechnet sich der Doppelbindungsanteils aus der Menge an Doppelbindungsequivalenten in der verwendeten Menge des HTNR bezogen auf einem Kilogramm Gesamtprodukt.

Die Berechnung des Butandiolanteils erfolgt durch die Menge an Butandiol ("BDO") mit einem Equivalentgewicht von 45 g bezogen auf einem Kilogramm Gesamtprodukt.

Tabelle 2a: Analog zu Tabelle 1a, nur wurden alle Werte auf eine Skala von 0 bis 1 skaliert, in dem der maximale Wert - Merkmalswert oder Prüfmerkmalswert - jeder Spalte (also jedes Deskriptors und jeder Verhaltenseigenschaft) bestimmt wurde und jeder einzelne Wert durch diesen maximalen Wert geteilt wurde.

Tabelle 3a: Analog zu Tabelle 1a, nur wurden alle Werte zunächst quadriert und dann auf eine Skala von 0 bis 1 skaliert, in dem der maximale Wert jeder Spalte (also jedes quadrierten Deskriptors und jeder quadrierten Verhaltenseigenschaft) bestimmt wurde und jeder einzelne quadrierte Merkmalswert oder Prüfmerkmalswert durch diesen maximalen quadrierten Wert geteilt wurde.

Tabelle 4a: Analog zu Tabelle 1a: Die Merkmalswerte wurden beibehalten und von den Prüfmerkmalswerten wurde der dekadische Logarithmus errechnet.

Tabelle 5a: Analog zu Tabelle 1a, dabei wurde zunächst das arithmetische Mittel sowie die Varianz der Merkmalswerte eines Deskriptors und der Prüfmerkmalswerte einer Verhaltenseigenschaft bestimmt. Das arithmetische Mittel ist die Summe aller Einzelwerte geteilt durch die Anzahl der Einzelwerte. Die Varianz wird berechnet aus einer Summe über alle Quadrate der Differenzen aus Einzelwerten minus deren arithmetischen Mittel, wobei die Summe dann geteilt wird durch die Anzahl der Einzelwerte. In Tabelle 5a werden nun Ergebnisse aus dem Quotienten aus Einzelwerte minus arithmetischen Mittel geteilt durch die Wurzel der Varianz angegeben (studentische Verteilung).

Tabelle 5a-2: Wie Tabelle 5a nur mit generisch benannten Deskriptoren M1- M6 und Verhaltenseigenschaften P1 - P5.

Tabelle A:

|  | IPDI | PCL | HTNR | BDO |
|---|---|---|---|---|
| Polyurethan 3 | 1,25 | 1,00 | 0,00 | 0,00 |
| Polyurethan 4 | 1,50 | 1,00 | 0,00 | 0,00 |
| Polyurethan 5 | 2,00 | 1,00 | 0,00 | 0,00 |
| Polyurethan 6 | 2,25 | 1,00 | 0,00 | 0,00 |
| Polyurethan 7 | 1,25 | 0,50 | 0,50 | 0,00 |
| Polyurethan 8 | 1,25 | 0.35 | 0,35 | 0,30 |
| Polyurethan 9 | 1,25 | 0,25 | 0,25 | 0,50 |

(fortgesetzt)

|  | IPDI | PCL | HTNR | BDO |
|---|---|---|---|---|
| Polyurethan 10 | 1,25 | 0,50 | 0,00 | 0,50 |
| Polyurethan 11 | 1,25 | 0,35 | 0,15 | 0,50 |
| Polyurethan 12 | 1,25 | 0,15 | 0,35 | 0,50 |
| Polyurethan 13 | 1,25 | 0,00 | 0,50 | 0,50 |

Tabelle B:

|  | Young Mod | TensileStr | EaB | TearStr | Shore A |
|---|---|---|---|---|---|
| Polyurethan 3 | 0,5 | 18,9 | 916 | 16,9 | 42 |
| Polyurethan 4 | 1,2 | 22,6 | 488 | 22,8 | 42 |
| Polyurethan 5 | 3,9 | 54,5 | 392 | 56,2 | 45 |
| Polyurethan 6 | 7,9 | 53,6 | 351 | 109,1 | 48 |
| Polyurethan 7 | 0,3 | 3,5 | 824 | 7,5 | 21 |
| Polyurethan 8 | 1,0 | 14,1 | 703 | 13,8 | 32 |
| Polyurethan 9 | 1,4 | 14,9 | 590 | 38,6 | 47 |
| Polyurethan 10 | 2,5 | 21,7 | 493 | 65,4 | 57 |
| Polyurethan 11 | 2,2 | 24,3 | 506 | 38,3 | 56 |
| Polyurethan 12 | 1,9 | 14,2 | 518 | 30,8 | 51 |
| Polyurethan 13 | 1,2 | 9,1 | 605 | 20,9 | 40 |

Table 1a

| Name | HardSeg | Urea | Urethane | Ester | Doublebond | BDO | YoungMod | TensileStr | EaB | TearStr | ShoreA |
|---|---|---|---|---|---|---|---|---|---|---|---|
| Polyurethane 3 | 34.37 | 0.31 | 2.48 | 5.08 | 0.00 | 0.00 | 0.50 | 18.90 | 916.00 | 16.90 | 42.00 |
| Polyurethane 4 | 38.59 | 0.58 | 2.32 | 4.75 | 0.00 | 0.00 | 1.20 | 22.60 | 488.00 | 22.80 | 42.00 |
| Polyurethane 5 | 45.59 | 1.03 | 2.05 | 4.21 | 0.00 | 0.00 | 3.90 | 54.60 | 392.00 | 56.20 | 45.00 |
| Polyurethane 6 | 48.52 | 1.21 | 1.94 | 3.98 | 0.00 | 0.00 | 7.90 | 53.60 | 351.00 | 109.10 | 48.00 |
| Polyurethane 7 | 19.93 | 0.18 | 1.44 | 1.47 | 16.88 | 0.00 | 0.30 | 3.50 | 824.00 | 7.50 | 21.00 |
| Polyurethane 8 | 28.07 | 0.23 | 1.84 | 1.32 | 15.16 | 0.55 | 1.00 | 14.10 | 703.00 | 13.80 | 32.00 |
| Polyurethane 9 | 36.65 | 0.28 | 2.27 | 1.17 | 13.35 | 1.14 | 1.40 | 14.90 | 590.00 | 38.60 | 47.00 |
| Polyurethane 10 | 54.90 | 0.43 | 3.40 | 3.49 | 0.00 | 1.70 | 2.50 | 21.70 | 493.00 | 65.40 | 57.00 |
| Polyurethane 11 | 42.27 | 0.33 | 2.62 | 1.88 | 9.24 | 1.31 | 2.20 | 24.30 | 506.00 | 38.30 | 56.00 |
| Polyurethane 12 | 32.35 | 0.25 | 2.01 | 0.62 | 16.50 | 1.00 | 1.90 | 14.20 | 518.00 | 30.80 | 51.00 |
| Polyurethane 13 | 27.51 | 0.21 | 1.71 | 0.00 | 20.04 | 0.85 | 1.20 | 9.10 | 605.00 | 20.00 | 40.00 |

Table 2a

| Name | HardSeg | Urea | Urethane | Ester | Doublebond | BDO | YoungMod | TensileStr | EaB | TearStr | ShoreA |
|---|---|---|---|---|---|---|---|---|---|---|---|
| Polyurethane 3 | 0.6260 | 0.2550 | 0.7277 | 1.0000 | 0.0000 | 0.0000 | 0.0633 | 0.3462 | 1.0000 | 0.1549 | 0.7368 |
| Polyurethane 4 | 0.7029 | 0.4773 | 0.6807 | 0.9358 | 0.0000 | 0.0000 | 0.1519 | 0.4139 | 0.5328 | 0.2090 | 0.7368 |
| Polyurethane 5 | 0.8304 | 0.8460 | 0.6031 | 0.8290 | 0.0000 | 0.0000 | 0.4937 | 1.0000 | 0.4279 | 0.5151 | 0.7895 |
| Polyurethane 6 | 0.8838 | 1.0000 | 0.5708 | 0.7845 | 0.0000 | 0.0000 | 1.0000 | 0.9817 | 0.3832 | 1.0000 | 0.8421 |
| Polyurethane 7 | 0.3630 | 0.1479 | 0.4219 | 0.2899 | 0.8423 | 0.0000 | 0.0380 | 0.0641 | 0.8996 | 0.0687 | 0.3684 |
| Polyurethane 8 | 0.5113 | 0.1898 | 0.5414 | 0.2606 | 0.7565 | 0.3235 | 0.1266 | 0.2582 | 0.7675 | 0.1265 | 0.5614 |
| Polyurethane 9 | 0.6676 | 0.2340 | 0.6677 | 0.2295 | 0.6662 | 0.6706 | 0.1772 | 0.2729 | 0.6441 | 0.3538 | 0.8246 |
| Polyurethane 10 | 1.0000 | 0.3505 | 1.0000 | 0.6872 | 0.0000 | 1.0000 | 0.3165 | 0.3974 | 0.5382 | 0.5995 | 1.0000 |
| Polyurethane 11 | 0.7699 | 0.2699 | 0.7700 | 0.3705 | 0.4610 | 0.7706 | 0.2785 | 0.4451 | 0.5524 | 0.3511 | 0.9825 |
| Polyurethane 12 | 0.5893 | 0.2065 | 0.5893 | 0.1215 | 0.8234 | 0.5882 | 0.2405 | 0.2601 | 0.5655 | 0.2823 | 0.8947 |
| Polyurethane 13 | 0.5011 | 0.1756 | 0.5012 | 0.0000 | 1.0000 | 0.5000 | 0.1519 | 0.1667 | 0.6605 | 0.1833 | 0.7018 |

Table 3a

| Name | HardSeg | Urea | Urethane | Ester | Doublebond | BDO | YoungMod | TensileStr | EaB | TearStr | ShoreA |
|---|---|---|---|---|---|---|---|---|---|---|---|
| Polyurethane 3 | 0.3919 | 0.0650 | 0.5295 | 1.0000 | 0.0000 | 0.0000 | 0.0040 | 0.1198 | 1.0000 | 0.0240 | 0.5429 |
| Polyurethane 4 | 0.4941 | 0.2278 | 0.4633 | 0.8757 | 0.0000 | 0.0000 | 0.0231 | 0.1713 | 0.2838 | 0.0437 | 0.5429 |
| Polyurethane 5 | 0.6896 | 0.7157 | 0.3637 | 0.6873 | 0.0000 | 0.0000 | 0.2437 | 1.0000 | 0.1831 | 0.2654 | 0.6233 |
| Polyurethane 6 | 0.7811 | 1.0000 | 0.3258 | 0.6155 | 0.0000 | 0.0000 | 1.0000 | 0.9637 | 0.1468 | 1.0000 | 0.7091 |
| Polyurethane 7 | 0.1318 | 0.0219 | 0.1780 | 0.0841 | 0.7095 | 0.0000 | 0.0014 | 0.0041 | 0.8092 | 0.0047 | 0.1357 |
| Polyurethane 8 | 0.2614 | 0.0360 | 0.2931 | 0.0679 | 0.5723 | 0.1047 | 0.0160 | 0.0667 | 0.5890 | 0.0160 | 0.3152 |
| Polyurethane 9 | 0.4457 | 0.0548 | 0.4459 | 0.0527 | 0.4438 | 0.4497 | 0.0314 | 0.0745 | 0.4149 | 0.1252 | 0.6799 |
| Polyurethane 10 | 1.0000 | 0.1228 | 1.0000 | 0.4723 | 0.0000 | 1.0000 | 0.1001 | 0.1580 | 0.2897 | 0.3593 | 1.0000 |
| Polyurethane 11 | 0.5928 | 0.0728 | 0.5929 | 0.1373 | 0.2125 | 0.5938 | 0.0776 | 0.1981 | 0.3051 | 0.1232 | 0.9652 |
| Polyurethane 12 | 0.3472 | 0.0426 | 0.3473 | 0.0148 | 0.6779 | 0.3460 | 0.0578 | 0.0676 | 0.3198 | 0.0797 | 0.8006 |
| Polyurethane 13 | 0.2511 | 0.0308 | 0.2512 | 0.0000 | 1.0000 | 0.2500 | 0.0231 | 0.0278 | 0.4362 | 0.0336 | 0.4925 |

Table 4a

| Name | HardSeg | Urea | Urethane | Ester | Doublebond | BDO | YoungMod | TensileStr | EaB | TearStr | ShoreA |
|---|---|---|---|---|---|---|---|---|---|---|---|
| Polyurethane 3 | 34.37 | 0.31 | 2.48 | 5.08 | 0.00 | 0.00 | -0.30 | 1.28 | 2.96 | 1.23 | 1.62 |
| Polyurethane 4 | 38.59 | 0.58 | 2.32 | 4.75 | 0.00 | 0.00 | 0.08 | 1.35 | 2.69 | 1.36 | 1.62 |
| Polyurethane 5 | 45.59 | 1.03 | 2.05 | 4.21 | 0.00 | 0.00 | 0.59 | 1.74 | 2.59 | 1.75 | 1.65 |
| Polyurethane 6 | 48.52 | 1.21 | 1.94 | 3.98 | 0.00 | 0.00 | 0.90 | 1.73 | 2.55 | 2.04 | 1.68 |
| Polyurethane 7 | 19.93 | 0.18 | 1.44 | 1.47 | 16.88 | 0.00 | -0.52 | 0.54 | 2.92 | 0.88 | 1.32 |
| Polyurethane 8 | 28.07 | 0.23 | 1.84 | 1.32 | 15.16 | 0.55 | 0.00 | 1.15 | 2.85 | 1.14 | 1.51 |
| Polyurethane 9 | 36.65 | 0.28 | 2.27 | 1.17 | 13.35 | 1.14 | 0.15 | 1.17 | 2.77 | 1.59 | 1.67 |
| Polyurethane 10 | 54.90 | 0.43 | 3.40 | 3.49 | 0.00 | 1.70 | 0.40 | 1.34 | 2.69 | 1.82 | 1.76 |
| Polyurethane 11 | 42.27 | 0.33 | 2.62 | 1.88 | 9.24 | 1.31 | 0.34 | 1.39 | 2.70 | 1.58 | 1.75 |
| Polyurethane 12 | 32.35 | 0.25 | 2.01 | 0.62 | 16.50 | 1.00 | 0.28 | 1.15 | 2.71 | 1.49 | 1.71 |
| Polyurethane 13 | 27.51 | 0.21 | 1.71 | 0.00 | 20.04 | 0.85 | 0.08 | 0.96 | 2.78 | 1.30 | 1.60 |

Table 5a

| Name | HardSeg | Urea | Urethane | Ester | Doublebond | BDO | YoungMod | TensileStr | EaB | TearStr | ShoreA |
|---|---|---|---|---|---|---|---|---|---|---|---|
| Polyurethane 3 | -0.2859 | -0.4478 | 0.5707 | 1.4816 | -1.0415 | -0.9837 | -0.8212 | -0.2506 | 2.0265 | -0.7546 | -0.1740 |
| Polyurethane 4 | 0.1467 | 0.3648 | 0.2536 | 1.2911 | -1.0415 | -0.9837 | -0.4794 | -0.0167 | -0.5591 | -0.5448 | -0.1740 |
| Polyurethane 5 | 0.8643 | 1.7129 | -0.2695 | 0.9742 | -1.0415 | -0.9837 | 0.8390 | 2.0069 | -1.1390 | 0.6424 | 0.1282 |
| Polyurethane 6 | 1.1646 | 2.2761 | -0.4874 | 0.8421 | -1.0415 | -0.9837 | 2.7921 | 1.9437 | -1.3867 | 2.5229 | 0.4304 |
| Polyurethane 7 | -1.7662 | -0.8396 | -1.4920 | -0.6257 | 1.0797 | -0.9837 | -0.9189 | -1.2245 | 1.4707 | -1.0887 | -2.2892 |
| Polyurethane 8 | -0.9317 | -0.6863 | -0.6856 | -0.7128 | 0.8636 | -0.0751 | -0.5771 | -0.5542 | 0.7397 | -0.8648 | -1.1813 |
| Polyurethane 9 | -0.0522 | -0.5246 | 0.1664 | -0.8052 | 0.6361 | 0.8996 | -0.3818 | -0.5036 | 0.0571 | 0.0168 | 0.3297 |
| Polyurethane 10 | 1.8186 | -0.0987 | 2.4075 | 0.5533 | -1.0415 | 1.8247 | 0.1554 | -0.0736 | -0.5289 | 0.9695 | 1.3369 |
| Polyurethane 11 | 0.5239 | -0.3936 | 0.8560 | -0.3867 | 0.1195 | 1.1805 | 0.0089 | 0.0908 | -0.4503 | 0.0061 | 1.2362 |
| Polyurethane 12 | -0.4930 | -0.6252 | -0.3626 | -1.1257 | 1.0319 | 0.6683 | -0.1376 | -0.5479 | -0.3778 | -0.2605 | 0.7326 |
| Polyurethane 13 | -0.9891 | -0.7381 | -0.9570 | -1.4862 | 1.4768 | 0.4205 | -0.4794 | -0.8704 | 0.1477 | -0.6444 | -0.3754 |

**Table 5a-2**

| Name | M1 | M2 | M3 | M4 | M5 | M6 | P1 | P2 | P3 | P4 | P5 |
|---|---|---|---|---|---|---|---|---|---|---|---|
| Polyurethane 3 | -0.2859 | -0.4478 | 0.5707 | 1.4816 | -1.0415 | -0.9837 | -0.8212 | -0.2506 | 2.0265 | -0.7546 | -0.1740 |
| Polyurethane 4 | 0.1467 | 0.3648 | 0.2536 | 1.2911 | -1.0415 | -0.9837 | -0.4794 | -0.0167 | -0.5591 | -0.5448 | -0.1740 |
| Polyurethane 5 | 0.8643 | 1.7129 | -0.2695 | 0.9742 | -1.0415 | -0.9837 | 0.8390 | 2.0069 | -1.1390 | 0.6424 | 0.1282 |
| Polyurethane 6 | 1.1646 | 2.2761 | -0.4874 | 0.8421 | -1.0415 | -0.9837 | 2.7921 | 1.9437 | -1.3867 | 2.5229 | 0.4304 |
| Polyurethane 7 | -1.7662 | -0.8396 | -1.4920 | -0.6257 | 1.0797 | -0.9837 | -0.9189 | -1.2245 | 1.4707 | -1.0887 | -2.2892 |
| Polyurethane 8 | -0.9317 | -0.6863 | -0.6856 | -0.7128 | 0.8636 | -0.0751 | -0.5771 | -0.5542 | 0.7397 | -0.8648 | -1.1813 |
| Polyurethane 9 | -0.0522 | -0.5246 | 0.1664 | -0.8052 | 0.6361 | 0.8996 | -0.3818 | -0.5036 | 0.0571 | 0.0168 | 0.3297 |
| Polyurethane 10 | 1.8186 | -0.0987 | 2.4075 | 0.5533 | -1.0415 | 1.8247 | 0.1554 | -0.0736 | -0.5289 | 0.9695 | 1.3369 |
| Polyurethane 11 | 0.5239 | -0.3936 | 0.8560 | -0.3867 | 0.1195 | 1.1805 | 0.0089 | 0.0908 | -0.4503 | 0.0061 | 1.2362 |
| Polyurethane 12 | -0.4930 | -0.6252 | -0.3626 | -1.1257 | 1.0319 | 0.6683 | -0.1376 | -0.5479 | -0.3778 | -0.2605 | 0.7326 |
| Polyurethane 13 | -0.9891 | -0.7381 | -0.9570 | -1.4862 | 1.4768 | 0.4205 | -0.4794 | -0.8704 | 0.1477 | -0.6444 | -0.3754 |

Tabelle 1b: Die Tabelle 1a wurde nun im csv-Fileformat in R eingelesen. Es wurde folgende R-Version verwendet: R version 3.4.3 (2017-11-30) - "Kite-Eating Tree", Copyright © 2017 The R Foundation for Statistical Computing, Platform: x86_64-w64-mingw32/x64 (64-bit). Anschließend wurde durch den Befehl "cor" die Pearson-Korrelationsmatrix - nachfolgend Korrelationsmatrix - errechnet. Für 11 Formulierungen ergibt sich mit df (degree of freedon) = 11-2 = 9 ein kritischer Pearson Korrelationswert von 0,602. Korrelationswerte >0,602 zeigen einen Pfeil nach oben (positive Korrelation), Korrelationswerte < -0,602 zeigen einen Pfeil nach unten (negative Korrelation), alle Werte dazwischen zeigen keine (statistische) Korrelation und damit einen horizontalen Pfeil.

Tabelle 2b: Korrelationsmatrix analog zu Tabelle 1b, nur wurde Tabelle 2a eingelesen. Die Tabelle ist identisches zu Tabelle 1b.

Tabelle 3b: Korrelationsmatrix analog zu Tabelle 1b, nur wurde Tabelle 3a eingelesen.

Tabelle 4b: Korrelationsmatrix analog zu Tabelle 1b, nur wurde Tabelle 4a eingelesen.

Tabelle 5b: Korrelationsmatrix analog zu Tabelle 1b, nur wurde Tabelle 5a eingelesen. Die Tabelle ist identisch zu Tabelle 1b.

Tabelle 5b-2: Wie Tabelle 5b nur mit generisch benannten Deskriptoren M1- M6 und Prüfmerkmalen P1 - P5.

Tabellen 1b, 2b, und 5b zeigen bis auf Rundungsfehler die gleichen Korrelationsergebnisse.

Es ist damit gezeigt, dass in ausgewählten Fällen der Abbildung aller Merkmalswerte aller Produktkompositionen sowie der Abbildung aller Prüfmerkmalswerte durch eine bijektive, stetige, streng monotone und wahlweise normierende Funktion identische Ergebnisse zeigen können; konkret in Tabelle 1a->2a mit einer Skalierung (=Normierung) und in Tabelle 1a->5a mit der Studentsche Verteilung.

Vergleicht man Tabelle 1b mit 3b so sieht man leichte Unterschiede in den Korrelationskoeffizienten, die in Vorzeichen und Größe sich ähneln, in einzelnen Fällen aber bei dem Kriterium der kritischen Pearson Korrelationswert zu einer anderen Bewertung führen, (vgl. z.B: c(Pearson) $_{Urea-Eab}$ = -0,694 (in Tabelle 1b) vs c(Pearson) $_{Urea-Eab}$ = -0,556 (in Tabelle 3b)). Dies ist durch die bessere Korrelation durch eine lineare Funktion gegenüber einer quadratischen Funktion zu erklären.

Es folgt, dass der bessere Pearson Korrelationswert in der Tabelle 1b darauf hinweist, dass eine geeignetere bijektive Abbildung die experimentellen Daten besser als in Tabelle 3b abbildet.

Auf ein analoges Beispiel sei mit c(Pearson) $_{Ester-TensileStr}$ = 0,600 (Tabelle 1b), c(Pearson) $_{Ester-TensileStr}$ = 0,457 (Tabelle 3b), c(Pearson) $_{Ester-TensileStr}$ = 0,619 (Tabelle 4b) hingewiesen. Hier zeigt sich, dass Tabelle 4b mit dem dekadischen Logarithmus als bijektive, stetige, streng monotone Funktion bessere Ergebnisse gefunden werden.

Somit werden die positiven und negativen Korrelation aus Tabelle 1b - 5b gemeinsam betrachtet und jeweils der maximale (für positive) bzw. der minimale (für negative) Korrelationswerte gewählt und gegen den kritischen Korrelationswert verglichen. So erhält man eine Gesamtsicht (hier in Tabelle 3), wobei deutlich wird, dass verschiedenen bijektive, stetige, streng monotone und wahlweise normierende Abbildungen oder Funktionen geprüft werden sollten. Dies kann durch grafische Darstellung der Daten unterstützt werden. Qualitativ erhält man so einen gute Übersicht, welche Deskriptoren mit welchen Verhaltenseigenschaften eine positive oder negative Korrelation aufweisen.

## Tabelle 1b

| | HardSeg | Urea | Urethane | Ester | Doublebond | BDO | YoungMod | TensileStr | EaB | TearStr | ShoreA |
|---|---|---|---|---|---|---|---|---|---|---|---|
| HardSeg | ⇑ 1.000 | ⇑ 0.650 | ⇑ 0.751 | ⇒ 0.566 | ⇓ -0.774 | ⇒ 0.281 | ⇑ 0.650 | ⇑ 0.711 | ⇓ -0.701 | ⇑ 0.808 | ⇑ 0.805 |
| Urea | ⇑ 0.650 | ⇑ 1.000 | ⇒ 0.025 | ⇑ 0.614 | ⇓ -0.693 | ⇒ -0.436 | ⇑ 0.887 | ⇑ 0.964 | ⇓ -0.694 | ⇑ 0.820 | ⇒ 0.272 |
| Urethane | ⇑ 0.751 | ⇒ 0.025 | ⇑ 1.000 | ⇒ 0.420 | ⇒ -0.577 | ⇒ 0.578 | ⇒ 0.035 | ⇒ 0.126 | ⇒ -0.212 | ⇒ 0.297 | ⇑ 0.751 |
| Ester | ⇒ 0.566 | ⇑ 0.614 | ⇒ 0.420 | ⇑ 1.000 | ⇓ -0.960 | ⇒ -0.490 | ⇒ 0.325 | ⇒ 0.600 | ⇒ -0.127 | ⇒ 0.363 | ⇒ 0.183 |
| Doublebond | ⇓ -0.774 | ⇓ -0.693 | ⇒ -0.577 | ⇓ -0.960 | ⇑ 1.000 | ⇒ 0.281 | ⇒ -0.470 | ⇓ -0.702 | ⇒ 0.335 | ⇒ -0.553 | ⇒ -0.413 |
| BDO | ⇒ 0.281 | ⇒ -0.436 | ⇒ 0.578 | ⇒ -0.490 | ⇒ 0.281 | ⇑ 1.000 | ⇒ -0.153 | ⇒ -0.326 | ⇒ -0.191 | ⇒ 0.059 | ⇑ 0.606 |
| YoungMod | ⇑ 0.650 | ⇑ 0.887 | ⇒ 0.035 | ⇒ 0.325 | ⇒ -0.470 | ⇒ -0.153 | ⇑ 1.000 | ⇑ 0.859 | ⇓ -0.739 | ⇑ 0.950 | ⇒ 0.408 |
| TensileStr | ⇑ 0.711 | ⇑ 0.964 | ⇒ 0.126 | ⇒ 0.600 | ⇓ -0.702 | ⇒ -0.326 | ⇑ 0.859 | ⇑ 1.000 | ⇓ -0.695 | ⇑ 0.803 | ⇒ 0.397 |
| EaB | ⇓ -0.701 | ⇓ -0.694 | ⇒ -0.212 | ⇒ -0.127 | ⇒ 0.335 | ⇒ -0.191 | ⇓ -0.739 | ⇓ -0.695 | ⇑ 1.000 | ⇓ -0.746 | ⇓ -0.620 |
| TearStr | ⇑ 0.808 | ⇑ 0.820 | ⇒ 0.297 | ⇒ 0.363 | ⇒ -0.553 | ⇒ 0.059 | ⇑ 0.950 | ⇑ 0.803 | ⇓ -0.746 | ⇑ 1.000 | ⇒ 0.570 |
| ShoreA | ⇑ 0.805 | ⇒ 0.272 | ⇑ 0.751 | ⇒ 0.183 | ⇒ -0.413 | ⇑ 0.606 | ⇒ 0.408 | ⇒ 0.397 | ⇓ -0.620 | ⇒ 0.570 | ⇑ 1.000 |

## Tabelle 2b

|  | HardSeg | Urea | Urethane | Ester | Doublebond | BDO | YoungMod | TensileStr | EaB | TearStr | ShoreA |
|---|---|---|---|---|---|---|---|---|---|---|---|
| HardSeg | 1.000 | 0.647 | 0.754 | 0.566 | -0.774 | 0.281 | 0.650 | 0.711 | -0.701 | 0.808 | 0.805 |
| Urea | 0.647 | 1.000 | 0.024 | 0.612 | -0.689 | -0.437 | 0.889 | 0.963 | -0.694 | 0.820 | 0.270 |
| Urethane | 0.754 | 0.024 | 1.000 | 0.420 | -0.578 | 0.578 | 0.038 | 0.129 | -0.214 | 0.300 | 0.751 |
| Ester | 0.566 | 0.612 | 0.420 | 1.000 | -0.960 | -0.490 | 0.325 | 0.600 | -0.128 | 0.363 | 0.183 |
| Doublebond | -0.774 | -0.689 | -0.578 | -0.960 | 1.000 | 0.281 | -0.470 | -0.702 | 0.336 | -0.553 | -0.413 |
| BDO | 0.281 | -0.437 | 0.578 | -0.490 | 0.281 | 1.000 | -0.153 | -0.326 | -0.191 | 0.059 | 0.606 |
| YoungMod | 0.650 | 0.889 | 0.038 | 0.325 | -0.470 | -0.153 | 1.000 | 0.859 | -0.739 | 0.950 | 0.408 |
| TensileStr | 0.711 | 0.963 | 0.129 | 0.600 | -0.702 | -0.326 | 0.859 | 1.000 | -0.695 | 0.802 | 0.397 |
| EaB | -0.701 | -0.694 | -0.214 | -0.128 | 0.336 | -0.191 | -0.739 | -0.695 | 1.000 | -0.746 | -0.620 |
| TearStr | 0.808 | 0.820 | 0.300 | 0.363 | -0.553 | 0.059 | 0.950 | 0.802 | -0.746 | 1.000 | 0.570 |
| ShoreA | 0.805 | 0.270 | 0.751 | 0.183 | -0.413 | 0.606 | 0.408 | 0.397 | -0.620 | 0.570 | 1.000 |

## Tabelle 3b

|  | HardSeg | Urea | Urethane | Ester | Doublebond | BDO | YoungMod | TensileStr | EaB | TearStr | ShoreA |
|---|---|---|---|---|---|---|---|---|---|---|---|
| HardSeg | 1.000 | 0.552 | 0.746 | 0.441 | -0.759 | 0.498 | 0.504 | 0.572 | -0.628 | 0.676 | 0.765 |
| Urea | 0.552 | 1.000 | -0.119 | 0.467 | -0.540 | -0.355 | 0.897 | 0.968 | -0.556 | 0.853 | 0.153 |
| Urethane | 0.746 | -0.119 | 1.000 | 0.307 | -0.574 | 0.784 | -0.106 | -0.077 | -0.190 | 0.125 | 0.740 |
| Ester | 0.441 | 0.467 | 0.307 | 1.000 | -0.839 | -0.334 | 0.267 | 0.457 | 0.038 | 0.274 | 0.091 |
| Doublebond | -0.759 | -0.540 | -0.574 | -0.839 | 1.000 | -0.027 | -0.388 | -0.563 | 0.249 | -0.468 | -0.444 |
| BDO | 0.498 | -0.355 | 0.784 | -0.334 | -0.027 | 1.000 | -0.216 | -0.309 | -0.292 | 0.009 | 0.731 |
| YoungMod | 0.504 | 0.897 | -0.106 | 0.267 | -0.388 | -0.216 | 1.000 | 0.795 | -0.488 | 0.966 | 0.210 |
| TensileStr | 0.572 | 0.968 | -0.077 | 0.457 | -0.563 | -0.309 | 0.795 | 1.000 | -0.553 | 0.757 | 0.203 |
| EaB | -0.628 | -0.556 | -0.190 | 0.038 | 0.249 | -0.292 | -0.488 | -0.553 | 1.000 | -0.546 | -0.598 |
| TearStr | 0.676 | 0.853 | 0.125 | 0.274 | -0.468 | 0.009 | 0.966 | 0.757 | -0.546 | 1.000 | 0.369 |
| ShoreA | 0.765 | 0.153 | 0.740 | 0.091 | -0.444 | 0.731 | 0.210 | 0.203 | -0.598 | 0.369 | 1.000 |

## Tabelle 4b

|  | HardSeg | Urea | Urethane | Ester | Doublebond | BDO | YoungMod | TensileStr | EaB | TearStr | ShoreA |
|---|---|---|---|---|---|---|---|---|---|---|---|
| HardSeg | 1.000 | 0.650 | 0.751 | 0.566 | -0.774 | 0.281 | 0.790 | 0.829 | -0.736 | 0.903 | 0.787 |
| Urea | 0.650 | 1.000 | 0.025 | 0.614 | -0.693 | -0.436 | 0.770 | 0.819 | -0.781 | 0.727 | 0.295 |
| Urethane | 0.751 | 0.025 | 1.000 | 0.420 | -0.577 | 0.578 | 0.270 | 0.407 | -0.204 | 0.484 | 0.714 |
| Ester | 0.566 | 0.614 | 0.420 | 1.000 | -0.960 | -0.490 | 0.207 | 0.619 | -0.224 | 0.328 | 0.202 |
| Doublebond | -0.774 | -0.693 | -0.577 | -0.960 | 1.000 | 0.281 | -0.426 | -0.756 | 0.421 | -0.558 | -0.421 |
| BDO | 0.281 | -0.436 | 0.578 | -0.490 | 0.281 | 1.000 | 0.182 | -0.087 | -0.100 | 0.267 | 0.554 |
| YoungMod | 0.790 | 0.770 | 0.270 | 0.207 | -0.426 | 0.182 | 1.000 | 0.836 | -0.943 | 0.943 | 0.711 |
| TensileStr | 0.829 | 0.819 | 0.407 | 0.619 | -0.756 | -0.087 | 0.836 | 1.000 | -0.760 | 0.833 | 0.700 |
| EaB | -0.736 | -0.781 | -0.204 | -0.224 | 0.421 | -0.100 | -0.943 | -0.760 | 1.000 | -0.866 | -0.614 |
| TearStr | 0.903 | 0.727 | 0.484 | 0.328 | -0.558 | 0.267 | 0.943 | 0.833 | -0.866 | 1.000 | 0.800 |
| ShoreA | 0.787 | 0.295 | 0.714 | 0.202 | -0.421 | 0.554 | 0.711 | 0.700 | -0.614 | 0.800 | 1.000 |

## Tabelle 5b

|  | HardSeg | Urea | Urethane | Ester | Doublebond | BDO | YoungMod | TensileStr | EaB | TearStr | ShoreA |
|---|---|---|---|---|---|---|---|---|---|---|---|
| HardSeg | 1.000 | 0.647 | 0.754 | 0.566 | -0.774 | 0.281 | 0.650 | 0.711 | -0.701 | 0.808 | 0.805 |
| Urea | 0.647 | 1.000 | 0.024 | 0.612 | -0.689 | -0.437 | 0.889 | 0.963 | -0.694 | 0.820 | 0.270 |
| Urethane | 0.754 | 0.024 | 1.000 | 0.420 | -0.578 | 0.578 | 0.038 | 0.129 | -0.214 | 0.300 | 0.751 |
| Ester | 0.566 | 0.612 | 0.420 | 1.000 | -0.960 | -0.490 | 0.325 | 0.600 | -0.128 | 0.363 | 0.183 |
| Doublebond | -0.774 | -0.689 | -0.578 | -0.960 | 1.000 | 0.281 | -0.470 | -0.702 | 0.336 | -0.553 | -0.413 |
| BDO | 0.281 | -0.437 | 0.578 | -0.490 | 0.281 | 1.000 | -0.153 | -0.326 | -0.191 | 0.059 | 0.606 |
| YoungMod | 0.650 | 0.889 | 0.038 | 0.325 | -0.470 | -0.153 | 1.000 | 0.859 | -0.739 | 0.950 | 0.408 |
| TensileStr | 0.711 | 0.963 | 0.129 | 0.600 | -0.702 | -0.326 | 0.859 | 1.000 | -0.695 | 0.803 | 0.397 |
| EaB | -0.701 | -0.694 | -0.214 | -0.128 | 0.336 | -0.191 | -0.739 | -0.695 | 1.000 | -0.746 | -0.620 |
| TearStr | 0.808 | 0.820 | 0.300 | 0.363 | -0.553 | 0.059 | 0.950 | 0.803 | -0.746 | 1.000 | 0.570 |
| ShoreA | 0.805 | 0.270 | 0.751 | 0.183 | -0.413 | 0.606 | 0.408 | 0.397 | -0.620 | 0.570 | 1.000 |

## Tabelle 5b-2

|  | M1 | M2 | M3 | M4 | M5 | M6 | P1 | P2 | P3 | P4 | P5 |
|---|---|---|---|---|---|---|---|---|---|---|---|
| M1 | 1.000 | 0.647 | 0.754 | 0.566 | -0.774 | 0.281 | 0.650 | 0.711 | -0.701 | 0.808 | 0.805 |
| M2 | 0.647 | 1.000 | 0.024 | 0.612 | -0.689 | -0.437 | 0.889 | 0.963 | -0.694 | 0.820 | 0.270 |
| M3 | 0.754 | 0.024 | 1.000 | 0.420 | -0.578 | 0.578 | 0.038 | 0.129 | -0.214 | 0.300 | 0.751 |
| M4 | 0.566 | 0.612 | 0.420 | 1.000 | -0.960 | -0.490 | 0.325 | 0.600 | -0.128 | 0.363 | 0.183 |
| M5 | -0.774 | -0.689 | -0.578 | -0.960 | 1.000 | 0.281 | -0.470 | -0.702 | 0.336 | -0.553 | -0.413 |
| M6 | 0.281 | -0.437 | 0.578 | -0.490 | 0.281 | 1.000 | -0.153 | -0.326 | -0.191 | 0.059 | 0.606 |
| P1 | 0.650 | 0.889 | 0.038 | 0.325 | -0.470 | -0.153 | 1.000 | 0.859 | -0.739 | 0.950 | 0.408 |
| P2 | 0.711 | 0.963 | 0.129 | 0.600 | -0.702 | -0.326 | 0.859 | 1.000 | -0.695 | 0.803 | 0.397 |
| P3 | -0.701 | -0.694 | -0.214 | -0.128 | 0.336 | -0.191 | -0.739 | -0.695 | 1.000 | -0.746 | -0.620 |
| P4 | 0.808 | 0.820 | 0.300 | 0.363 | -0.553 | 0.059 | 0.950 | 0.803 | -0.746 | 1.000 | 0.570 |
| P5 | 0.805 | 0.270 | 0.751 | 0.183 | -0.413 | 0.606 | 0.408 | 0.397 | -0.620 | 0.570 | 1.000 |

Tabelle 3

| | YoungMod | TensileStr | EaB | TearStr | ShoreA |
|---|---|---|---|---|---|
| HardSeg | ⬆ 0.790 | ⬆ 0.829 | ⬇ -0.736 | ⬆ 0.903 | ⬆ 0.805 |
| Urea | ⬆ 0.897 | ⬆ 0.968 | ⬇ -0.781 | ⬆ 0.853 | ➡ 0.295 |
| Urethane | ➡ 0.270 | ➡ 0.407 | ➡ -0.214 | ➡ 0.484 | ⬆ 0.751 |
| Ester | ➡ 0.325 | ⬆ 0.619 | ➡ 0.038 | ➡ 0.363 | ➡ 0.202 |
| Doublebond | ➡ -0.470 | ⬇ -0.756 | ➡ 0.421 | ➡ -0.558 | ➡ -0.444 |
| BDO | ➡ 0.182 | ➡ -0.326 | ➡ -0.292 | ➡ 0.267 | ⬆ 0.731 |

Aus Tabelle 3 ergeben sich somit folgende Schlüsse:

- das Elastizitätmodul ("YoungMod") als Verhaltenseigenschaft wird vom Hardsegmentanteil ("HardSeg") und dem Harnstoffanteil ("Urea") -jeweils Deskriptoren - bestimmt. Da beide eine positive Korrelation aufweisen bedeutet hier ein höheres Hardsegment und ein höherer Harnstoffanteill ein höheres Elastizitätsmodul.
- Gleiches gilt für die Verhaltenseigenschaft Bruchkraft ("TensileStr"), die positiv mit dem Deskriptor Esteranteil zusätzlich korreliert, während der Deskriptor Doppelbindungsanteil ("Doublebond") negativ korreliert.
- Die Verhaltenseigenschaft Bruchdehnung ("EaB") zeigt antiproportionales Verhalten wie das Elastizitätsmodul.
- Die Verhaltenseigenschaft Reißfestigkeit ("TearStr") ist dem Elastizitätmodul korreliert.
- Die Verhaltenseigenschaft Shore A Härte ("Shore A") korreliert positiv mit dem Hardsegmentanteil ("HardSeg"), dem Urethananteil ("Urethane") und dem Deskriptor Butandiolanteil ("BDO") und ist damit die einzige betrachtete Verhaltenseigenschaft, die von dem Deskriptor "BDO" abhängt.

Hiermit ergeben sich folgende Abhängigkeiten, die eine Optimierung der Verhaltenseigenschaften bedingen.

- das Elastizitätmodul ("YoungMod"), die Bruchdehnung ("EaB") und die Reißfestigkeit ("TensileStr") bedingen einander.
- Die Reißfestigkeit ("TensileStr") läßt sich durch hohen Estergehalt und niedrigen Doppelbindungsanteil erhöhen (bzw. auch umgekehrt erniedrigen).
- Die Shore A Härte ("Shore A") wird durch hohen Urethananteil und hohen Butandiolanteil erhöht.

Die Abhängigkeiten der Verhaltenseigenschaften zu den Deskriptoren sind durch Auswertung der Korrelationsmatrix verstanden und können nun auf Verhaltenseigenschaften bezogene Ziel-Prüfmerkmalswertn gemäß einem Ziel-Anforderungsprofil eines Ziel-Mischungsprodukts, beispielsweise in einer Anwendung in Lacken, Klebstoffen, Dichtmassen, Gießharze und Schäumen adaptiert werden. Geeignete Kombinationen von Verhaltenseigenschaften lassen sich aus den Datensätzen direkt entnehmen oder auch extrapolieren.

Auf die beschriebene Weise ermöglicht das vorschlagsgemäße Verfahren Produktentwicklungen systematisch durchzuführen und erlaubt es dem Entwickler von Lacken, Klebstoffen, Dichtmassen, Gießharze und Schäumen, ein zielgerichtes Vorgehen zu finden und klare Handlungsanweisungen zur Verbesserung dieser Produkte abzuleiten. Weiterhin wurde gezeigt, dass Verhaltenseigenschaften von bestimmten (einzelnen oder mehreren) Deskriptoren abhängig sind, was einen Wissensaufbau für zukünftige Aufgaben erlaubt.

Betrachtet man vergleichend die Tabellen 5a und 5a-2 sowie Tabellen 5b und 5b-2 so sind dort die Deskriptoren als M1-M6 und die Verhaltenseigenschaften als P1-P5 in den Tabellen 5a-2 und 5b-2 genannt. Der Inhalt der Korrelationsmatrices in Tabellen 5b und 5b-2 ist identisch, so sodass gezeigt ist, dass bei einer zusätzlichen Kodierung der Deskriptoren und der Verhaltenseigenschaften und der Verwendung von normierenden Abbildungen (hier z.B. die studentische Verteilung) weder die Zuordnung zu bestimmten Größen bekannt ist, noch um was für eine Größe es sich dabei handelt. Auch durch die Zahlenwerte, die durch die bijektive Abbildung verändert wurden, entfällt somit die Möglichkeit für den Fachmann die Korrelationszusammenhänge auf die Deskriptoren bzw. die Verhaltenseigenschaften abzuleiten. Der Inhalt der Korrelationsmatrix als Handlungsanweisungen für die jeweilige Seite (die entweder nur die Deskriptoren kennt oder die andererseits nur die Verhaltenseigenschaften kennt) bleibt aber erhalten.

Im Folgenden soll gezeigt werden, dass mit diesem Vorgehen rational ableitbare Vorschläge für neue Produktverteilungen der Produktkompositionen der in Tabelle 1 angegeben Beispielen errechnet werden können. Dazu sei zunächst auf Tabelle 4 verwiesen, in der die Gewichtsverhältnisse der Produktverteilungen aus Tabelle 1 angegeben sind.

EP 3 561 702 A1

Tabelle 4

|  | IPDI | PCL | HTNR | BDO |
|---|---|---|---|---|
| Polyurethan 3 | 34.16 | 65.84 | 0.00 | 0.00 |
| Polyurethan 4 | 38.37 | 61.63 | 0.00 | 0.00 |
| Polyurethan 5 | 45.36 | 54.64 | 0.00 | 0.00 |
| Polyurethan 6 | 48.29 | 51.71 | 0.00 | 0.00 |
| Polyurethan 7 | 19.78 | 19.06 | 61.15 | 0.00 |
| Polyurethan 8 | 25.40 | 17.14 | 54.97 | 2.49 |
| Polyurethan 9 | 31.34 | 15.10 | 48.43 | 5.13 |
| Polyurethan 10 | 47.01 | 45.30 | 0.00 | 7.69 |
| Polyurethan 11 | 36.16 | 24.39 | 33.53 | 5.92 |
| Polyurethan 12 | 27.65 | 7.99 | 59.83 | 4.52 |
| Polyurethan 13 | 23.50 | 0.00 | 72.65 | 3.85 |

Für die Berechnung der Tabelle 4 aus Tabelle 1, wurden die Equivalentgewichte der vier Komponenten IPDI: 110 g/mol, PCL: 265 g/mol, HTNR 850 g/mol und BDO 45 g/mol verwendet. Diese wurde mit den in Tabelle 1 angegeben Equivalentangaben multipliziert und dann auf Prozent umgerechnet.

Wie schon aus Tabelle 3 abgeleitet sind die wichtigen mechanischen Eigenschaften Elastizitätsmoduls und Bruchdehnung typische zu optimierende Verhaltenseigenschaften. Der Deskriptor Harnstoffgehalt und der Deskriptor Hartsegmentanteil bestimmen beide Verhaltenseigenschaften - nur die Verhaltenseigenschaft Elastizitätsmoduls eben in umgekehrter Weise wie die Verhaltenseigenschaft Bruchdehnung. In der Regel stellt eine derartige Entwicklungsaufgabe ein Problem für den Entwickler dar, da er vermeintlich ausschließlich einen Kompromiss suchen kann.

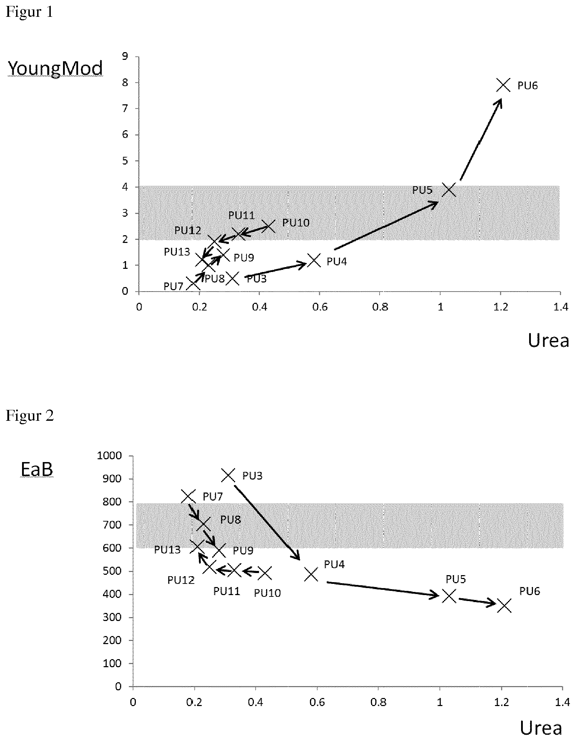

Um nun eine Verbesserung beider Verhaltenseigenschaften zu erreichen, eignet sich in einer multivariate Analyse besonders eine grafische Darstellung. Dabei kann es wichtig sein, die Identifikation der Deskriptoren (hier der Harnstoffgehalt "Urea") als wichtigen Einflußfaktor auf diese beiden Verhaltenseigenschaften genauer zu betrachten. So zeigt Figur 1 nun das Elastizitätmodul ("YoungMod") aufgetragen gegen den Harnstoffgehalt ("Urea") während Figur 2 die Bruchdehnung ("EaB") aufgetragen gegen den Harnstoffgehalt ("Urea") zeigt. Beide Graphen zeigen einen grau markierten vorgegebenen Zielbereich, für den eine neue Produktverteilung der Produktkomposition aus IPDI/PCL/HTNR/BDO gesucht ist und welcher somit ein Ziel-Anforderungsprofil für das gesuchte Ziel-Mischungsprodukt darstellt.

Durch Vergleich der in den grauen Zielbereich sich befindenden Produktkompositionen/Produktverteilungen wird deutlich, dass kein Produkt aus der Auswahl PU3 bis PU13 das durch beide Ziel-Verhaltenseigenschaften beschriebene Ziel-Anforderungsprofil erfüllt.

Figur 1 zeigt die starke positive Korrelation des Harnstoffgehalts ("Urea") mit dem Elastizitätsmodul ("YoungMod") z.B. an der Beispielreihe PU3 bis PU6 (PU= Polyurethan). Dies wurde in dieser Beispielsreihe durch einen zunehmende Equivalentüberschuß von IPDI - dargestellt durch die Vektoren von PU3 bis PU6 - erreicht, allerdings unter Verlust der Bruchdehnung ("EaB"), wie Figur 2 deutlich zeigt. Gleiches gilt für die Beispielreihe PU7-PU9, wo durch einen zunehmend höheren BDO Einsatz, der durch das niedrige Equivalentverhältnis ebenfalls zu einem höheren Harnstoffgehalt führt, ebenfalls ein höheres Elastizitätsmodul - aber auf einem niedrigerem Niveau - erhalten wird, das dann auch zu einem Verlust in der Bruchdehnung ("EaB") führt.

Unter Verwendung der gleichen Rohstoffe, bietet sich nun PU10 als Ausgangpunkt an, da dieser im Elastizitätsmodulzielbereich bereits liegt, benötigt wird nun aber noch eine Erhöhung der Bruchdehnung. Die Veränderung des Harnstoffgehalts von PU3 nach PU4 führt zu einem geringen Gewinn im Elastizitätsmodul und großen Verlust in der Bruchdehnung. Daher sollte das umgekehrte Vorgehen einen geringen Verlust im Elastizitätsmodul und einen großen Gewinn an Bruchdehnung ergeben. Auf diese Weise wird ein Ziel-Deskriptorenprofil erhalten. Ausgehend von der PU10 sollte das in den Zielbereich führen.

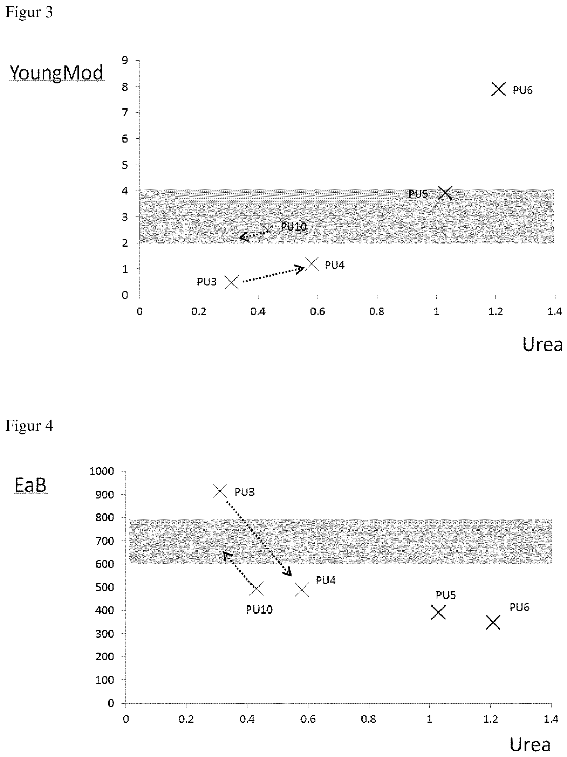

Aus diesem Deskriptorenprofil wiederum kann auf die Produktkomposition geschlossen werden. Die Veränderung der Produktverteilung von PU4 nach PU3 ist die Reduktions des Equivalentverhältnisses von Isocyanat zu Alkoholen. Als Handlungsanweisung folgt, das ausgehend von der Produktkomposition PU10, das Equivalentverhältnis weiter reduziert werden sollte und zwar grafisch abgeschätzt anhand der Identifikation des Ziel-Merkmalswertes des Deskriptors Harnstoffgehalts "Urea", oder anders ausgedrückt wird die Korrelationsmatrix zur Änderung der Verhaltenseigenschaften genutzt, um über die Deskriptoren zu einer Ziel-Produktkomposition des Ziel-Mischungsprodukts zu kommen. Grafisch ist dies nun in Figur 3 und 4 gezeigt, wobei die Projektion der Vektoren in die gleiche Richtung der Verhaltenseigenschaftsveränderung von PU3 nach PU4 verwendet wird, aber in Richtung invertiert und in Länge ungefähr halbiert wurde, um im grau hinterlegten Zielbereich zu landen. Damit ergibt sich auch schon die neue Zielkomposition basierend auf PU10, die in Tabelle A2 gezeigt wird. Polyurethan 3, 4 und 10 sind als Referenz erneut angegeben, das neue Ziel-Mischungsprodukt Polyurethane 10-Neu zeigt die Ziel-Verhaltenseigenschafteigenschaft mit einem graphisch abgeschätzen Harnstoffgehalt von 0,33. Das Equivalentverhältnis wird nun derart kalkuliert, dass der Zielharnstoffgehalt erreicht wird, so daß die Projektion der Vektoren in Figur 6 noch in den grauhinterlegten Zielbereich führt. Das Equivalentverhältnis von IPDI wird somit auf 1.189 gewählt (was einen Harnstoffgehalt von 0,33 ergibt) und wie oben für Tabelle 4 gezeigt und erläutert wurde, die neue Zielverteilung berechnet (siehe Tabelle 4-Neu).

Tabelle A2: Ableitung der neuen Equivalentverhältnisse für die neue Zielkomposition Polyurethan 10-Neu (siehe auch Figur 6 und 7)

|  | IPDI | PCL | HTNR | BDO |
|---|---|---|---|---|
| Polyurethan 3 | 1,25 | 1,00 | 0,00 | 0,00 |
| Polyurethan 4 | 1,50 | 1,00 | 0,00 | 0,00 |
| Polyurethan 10 | 1,25 | 0,50 | 0,00 | 0,50 |
| Polyurethan 10-Neu | 1,19 | 0,50 | 0,00 | 0,50 |

Tabelle 4-Neu: Ergebnis der neuen Produktverteilung von Polyurethane 10-Neu

|  | IPDI | PCL | HTNR | BDO |
|---|---|---|---|---|
| Polyurethan 10-Neu | 45.76 | 46.36 | 0.00 | 7.87 |

Er konnte somit gezeigt werden, dass eine grafische Analyse der Verhaltenseigenschaftsveränderungen in Bezug auf zuvor statistisch als relevant identifizierte Deskriptoren ein geeignetes Vorgehen ist, neue Produktkompositionen und deren Produktverteilungen vorherzusagen. Die Begrifflichkeit der Projektion leitet sich dabei von den Vektoren ab, die sich aus der grafischen Betrachtung im Dimensionsraum Verhaltenseigenschaft zu Deskriptoren ergibt.

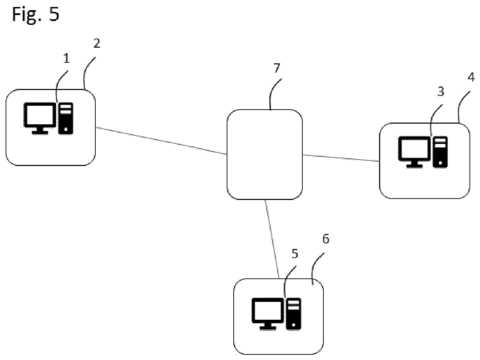

Fig. 5 zeigt ein erstes 1, ein zweites 3 und ein drittes 5 Computersystem zur Ausführung des vorschlagsgemäßen Verfahrens zum Ermitteln einer Produktkomposition für ein chemisches Mischungsprodukt. Dabei befindet sich das erste Computersystem 1 in einem eigenen Intranet 2, das zweite Computersystem 3 in ebenfalls einem eigenen Intranet 4 und ebenso das dritte Computersystem 5 in einem eigenen Intranet 6, wobei jedes Intranet 2, 4, 6 zu den jeweils andere disjunkt ist. Die drei zueinander disjunkten Intranets 2, 4, 6 können auch jeweils als erstes Intranet 2, als zweites Intranet 4 und als dritter Intranet 6 bezeichnet werden. Alle drei Computersysteme 1,3,5 sind verbunden durch das generelle Internet 7, wobei technische Schutzmassnahmen existieren, die einen Austausch von Informationen (insbesondere: abgebildete Merkmalswerte und abgebildete Prüfmerkmalswerte) zwischen Lieferant und Abnehmer nur durch deren spezielle Zugangsfreigabe möglich gemacht wird.

**Patentansprüche**

1. Verfahren zum Ermitteln einer Produktkomposition für ein chemisches Mischungsprodukt, wobei bei einer Vielzahl von ersten Produktkompositionen für ein jeweiliges chemisches Mischungsprodukt, wobei jede erste Produktkomposition durch eine numerische Produktverteilung zur Beschreibung von Anteilen von Komponenten der ersten Produktkomposition charakterisiert wird, für jede erste Produktkomposition der Vielzahl eine Reihe von Merkmalswerten, welche Merkmalswerte jeweils einen Deskriptor des jeweiligen Mischungsprodukts numerisch beschreiben, bereitgestellt wird, wobei die Reihe von Merkmalswerten für jedes Mischungsprodukt durch eine erste bijektive Abbildung auf eine Reihe von abgebildeten Merkmalswerten abgebildet wird, wobei für eine Vielzahl von zweiten Produktkompositionen für ein jeweiliges chemisches Mischungsprodukt eine Reihe von Prüfmerkmalswerten, welche Prüfmerkmalswerte jeweils eine Verhaltenseigenschaft des jeweiligen Mischungsprodukts numerisch beschreiben, bereitgestellt wird, wobei die Reihe von Prüfmerkmalswerten für jedes Mischungsprodukt durch eine zweite bijektive Abbildung auf eine Reihe von abgebildeten Prüfmerkmalswerten abgebildet wird, wobei zumindest die erste oder die zweite Abbildung eine Veränderung umfasst, wobei jede Reihe von abgebildeten Prüfmerkmalswerten einer zweiten Produktkomposition einer Reihe von veränderten Merkmalswerten einer ersten Produktkomposition zugeordnet wird, wobei durch eine multivariate Analyse der zugeordneten Reihen eine Korrelationsmatrix ermittelt wird, wobei ein Ziel-Anforderungsprofil zur Beschreibung mindestens einer Verhaltenseigenschaft eines Ziel-Mischungsprodukts vorgegeben wird und basierend auf dem Ziel-Anforderungsprofil sowie der Korrelationsmatrix ein Ziel-Deskriptorenprofil zur Beschreibung von Deskriptoren einer Ziel-Produktkomposition bestimmt wird.

2. Verfahren nach Anspruch 1, **dadurch gekennzeichnet, dass** das Bestimmen des Ziel-Deskriptorenprofils umfasst, basierend auf einem Vergleich des Ziel-Deskriptorenprofils mit den Merkmalswerten der ersten Produktkompositionen der Vielzahl eine erste Produktkomposition der Vielzahl als Ausgangs-Produktkomposition zu bestimmen und die Produktverteilung der Ausgangs-Produktkomposition basierend auf den Merkmalswerten der übrigen ersten Produktkompositionen der Vielzahl zum Erhalten der Ziel-Produktkomposition zu verändern.

3. Verfahren nach Anspruch 1 oder 2, **dadurch gekennzeichnet, dass** die Reihe von veränderten Prüfmerkmalswerten einer ersten Produktkomposition derjenigen Reihe von veränderten Merkmalswerten einer zweiten Produktkomposition zugeordnet wird, bei der die zweite Produktkomposition im Wesentlichen identisch zu der ersten Produktkomposition ist.

4. Verfahren nach einem der Ansprüche 1 bis 3, **dadurch gekennzeichnet, dass** die Reihe von Merkmalswerten für jede erste Produktkomposition auf einem ersten Computersystem (1) bereitgestellt wird, auf welchem die erste bijektive Abbildung ausgeführt wird, dass die Reihe von Prüfmerkmalswerten für jede zweite Produktkomposition auf einem zweiten Computersystem (3) bereitgestellt wird, auf welchem die zweite bijektive Abbildung ausgeführt wird und dass das erste Computersystem (1) und das zweite Computersystem (3) von einem jeweils disjunkten Intranet (2, 4) umfasst sind, vorzugsweise, dass die Bestimmung des Ziel-Deskriptorenprofils zumindest teilweise auf dem ersten Computersystem (1) durchgeführt wird.

5. Verfahren nach Anspruch 4, **dadurch gekennzeichnet, dass** die multivariate Analyse auf einem dritten Computersystem (5) ausgeführt wird, welches von einem Intranet (6) umfasst ist, dass zu dem jeweiligen Intranet (2, 4) des ersten Computersystems (1) und des zweiten Computersystems (3) disjunkt ist.

6. Verfahren nach Anspruch 4 oder 5, **dadurch gekennzeichnet, dass** die Produktverteilung und die Reihe von Merkmalswerten für jede erste Produktkomposition in dem ersten Computersystem (1) gegenüber dem zweiten Computersystem (3) datentechnisch abgekapselt wird und dass die Reihe von Prüfmerkmalswerten für jede zweite Produktkomposition in dem zweiten Computersystem (3) gegenüber dem ersten Computersystem (1) datentechnisch abgekapselt wird.

7. Verfahren nach einem der Ansprüche 4 bis 6, **dadurch gekennzeichnet, dass** die Reihe von veränderten Merkmalswerten für jede erste Produktkomposition von dem ersten Computersystem (1) an ein Ziel-Computersystem eines disjunkten Intranets, vorzugsweise an das zweite Computersystem (3) oder das dritte Computersystem (5), übertragen wird.

8. Verfahren nach einem der Ansprüche 1 bis 7, **dadurch gekennzeichnet, dass**, vorzugsweise in dem ersten Computersystem (1), ein Berechnungsmodell bereitgestellt ist, welches auf Eingabe von Merkmalswerten zur Beschreibung eines jeweiligen Deskriptors eine Produktverteilung einer Produktkomposition für ein Mischungsprodukt zur Approximation der Merkmalswerte ausgibt und dass, vorzugsweise in dem ersten Computersystem (1), das Ziel-Deskriptorenprofil dem Berechnungsmodell zur Ausgabe der Ziel-Produktkomposition eingegeben wird.

9. Verfahren nach Anspruch 8, **dadurch gekennzeichnet, dass** das Berechnungsmodell zumindest teilweise durch, vorzugsweise in dem ersten Computersystem (1) ausgeführte, multivariate Analyse der Reihe von Merkmalswerten jeder ersten Produktkomposition gegenüber der Produktverteilung dieser Produktkomposition ermittelt wird.

10. Verfahren nach einem der Ansprüche 1 bis 9, **dadurch gekennzeichnet, dass** für jede erste Produktkomposition der Vielzahl die Reihe von Merkmalswerten zumindest teilweise, vollzugsweise vollständig, durch eine Berechnung basierend auf der entsprechend Produktverteilung ermittelt wird, vorzugsweise, dass die Berechnung auf einem physikalischen Rechenmodell beruht, welches auf der Produktverteilung basiert.

11. Verfahren nach einem der Ansprüche 1 bis 10, **dadurch gekennzeichnet, dass** die erste bijektive Abbildung eine Koordinatentransformation von der Reihe von Merkmalswerten zu der Reihe von veränderten Merkmalswerten umfasst und/oder dass die zweite bijektive Abbildung eine Koordinatentransformation von der Reihe von Prüfmerkmalswerten zu der Reihe von veränderten Prüfmerkmalswerten umfasst.

12. Verfahren nach einem der Ansprüche 1 bis 11, **dadurch gekennzeichnet, dass** die erste bijektive Abbildung eine eindimensionale bijektive Sub-Abbildung für jeden einzelnen Deskriptor umfasst, vorzugsweise, dass die eindimensionale bijektive Sub-Abbildung jeden Deskriptors für jede erste Produktkomposition gleich ist.

13. Verfahren nach einem der Ansprüche 1 bis 12, **dadurch gekennzeichnet, dass** die erste bijektive Abbildung und/oder die zweite bijektive Abbildung eine stetige und streng monotone Funktion mit sich kontinuierlich verändernder Ableitung ist.

14. Verfahren zur Herstellung eines chemischen Mischungsprodukts aus einer Produktkomposition, **dadurch gekennzeichnet, dass** die Produktkomposition nach dem Verfahren nach einem der Ansprüche 1 bis 14 ermittelt wurde.

15. Chemisches Mischungsprodukt, **dadurch gekennzeichnet, dass** das chemische Mischungsprodukt durch das Verfahren nach Anspruch 14 hergestellt wurde.

Figur 1

Figur 2

Figur 3

Figur 4

Fig. 5

**Europäisches Patentamt**
**European Patent Office**
**Office européen des brevets**

# EUROPÄISCHER RECHERCHENBERICHT

Nummer der Anmeldung

EP 18 16 8734

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2009/020772 A2 (DOW GLOBAL TECHNOLOGIES INC [US]; DRABISH JEFFREY [US]; PARRISH JOHN []) 12. Februar 2009 (2009-02-12) * Seite 3 - Seite 7; Abbildungen 1-3 * | 1-15 | INV. G06F19/00 C08J3/00 |
| X | EP 1 785 897 A1 (AVANTIUM INT BV [NL]) 16. Mai 2007 (2007-05-16) | 14,15 | |
| A | * Spalte 9 - Spalte 20; Ansprüche 1-27 * | 1-13 | |
| X | WO 02/077772 A2 (TRANSFORM PHARMACEUTICALS INC [US]) 3. Oktober 2002 (2002-10-03) | 14,15 | |
| A | * Ansprüche 11-27; Abbildungen 1-6 * | 1-13 | |
| X | Alessandra Martins Coelho ET AL: "Methodology for Optimization of Polymer Blends Composition" In: "Principal Component Analysis - Engineering Applications", 7. März 2012 (2012-03-07), InTech, XP055503344, ISBN: 978-953-51-0182-6 DOI: 10.5772/37504, | 14,15 | |
| A | * das ganze Dokument * | 1-13 | |

| | | | RECHERCHIERTE SACHGEBIETE (IPC) |
|---|---|---|---|
| | | | G06F C08J |

Der vorliegende Recherchenbericht wurde für alle Patentansprüche erstellt

| Recherchenort | Abschlußdatum der Recherche | Prüfer |
|---|---|---|
| Den Haag | 30. August 2018 | Schmidt-Yodlee, H |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 18 16 8734

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

30-08-2018

| Im Recherchenbericht angeführtes Patentdokument | Datum der Veröffentlichung | Mitglied(er) der Patentfamilie | Datum der Veröffentlichung |
|---|---|---|---|
| WO 2009020772 A2 | 12-02-2009 | AR 067828 A1 | 21-10-2009 |
| | | BR PI0813613 A2 | 30-12-2014 |
| | | CN 102124467 A | 13-07-2011 |
| | | EP 2188751 A1 | 26-05-2010 |
| | | JP 5778927 B2 | 16-09-2015 |
| | | JP 2010535894 A | 25-11-2010 |
| | | SG 183700 A1 | 27-09-2012 |
| | | US 2010209307 A1 | 19-08-2010 |
| | | WO 2009020772 A2 | 12-02-2009 |
| EP 1785897 A1 | 16-05-2007 | EP 1785897 A1 | 16-05-2007 |
| | | EP 1785898 A1 | 16-05-2007 |
| | | US 2007106477 A1 | 10-05-2007 |
| | | US 2007112527 A1 | 17-05-2007 |
| WO 02077772 A2 | 03-10-2002 | AU 2002305092 A1 | 08-10-2002 |
| | | CA 2441931 A1 | 03-10-2002 |
| | | EP 1381857 A2 | 21-01-2004 |
| | | WO 02077772 A2 | 03-10-2002 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- Some extension of Student's t and Pearson's r central distributions, Technical Report. **A. L. SOCKLOGG ; J. N EDNEY.** Measurement and Research Center. Temple University, Mai 1972

- **G. H. DUNTEMAN.** Principal Component Analysis. Sage Publications, 1989
- **W. PANWIRIYARAT et al.** *J. Polym. Environ.,* 2013, vol. 21, 807-815